# EUROPÄISCHE PATENTSCHRIFT

(11) **EP 2 688 473 B1**
(45) Veröffentlichungstag und Bekanntmachung des Hinweises auf die Patenterteilung: **14.01.2015**
(21) Anmeldenummer: 12710081.6
(22) Anmeldetag: 21.03.2012
(51) Int. Cl.: A61B 5/145, A61B 5/1455, A61B 5/1459, A61B 5/151, A61M 5/32

(54) **ANALYTISCHES HILFSMITTEL MIT HYDROPHILER BESCHICHTUNG ENTHALTEND NANOPARTIKEL MIT SILICIUMDIOXIDSTRUKTUR**
ANALYTICAL AID WITH HYDROPHILIC COATING THAT CONTAINS NANOPARTICLES WITH A SILICON DIOXIDE STRUCTURE
MOYEN AUXILIAIRE D'ANALYSE À REVÊTEMENT HYDROPHILE CONTENANT DES NANOPARTICULES À STRUCTURE DE DIOXYDE DE SILICIUM

(30) Priorität: 22.03.2011 EP 11159172
(43) Veröffentlichungstag der Anmeldung: 29.01.2014
(73) Patentinhaber: Roche Diagnostics GmbH, 68305 Mannheim (DE); F.Hoffmann-La Roche AG, 4070 Basel (CH)
(72) Erfinder: GREIWE, Peter, 69115 Heidelberg (DE); BABIC, Branislav, 68167 Mannheim (DE)
(74) Vertreter: Stößel, Matthias
(86) Internationale Anmeldenummer: PCT/EP2012/054993
(87) Internationale Veröffentlichungsnummer: WO 2012/126945

(56) Entgegenhaltungen:
- EP-A1- 1 887 355
- EP-A1- 1 894 525
- WO-A1-2010/045247
- US-A1- 2007 179 373
- US-A1- 2008 103 415

## Beschreibung

### Gebiet der Erfindung

Die vorliegende Erfindung betrifft ein analytisches Hilfsmittel, umfassend eine zumindest teilweise mit einer hydrophilen Beschichtung beschichtete Oberfläche, wobei die hydrophile Beschichtung Nanopartikel mit Siliciumdioxidstruktur und einer mittleren Partikelgröße bestimmt gemäß DIN ISO 22412:2008 (dynamische Lichtstreuung), im Bereich von 1 bis 500 nm enthält. Weiterhin betrifft die vorliegende Erfindung ein Verfahren zur Herstellung eines analytischen Hilfsmittels, umfassend eine zumindest teilweise mit einer hydrophilen Beschichtung beschichtete Oberfläche enthaltend Nanopartikel mit Siliciumdioxidstruktur und einer mittleren Partikelgröße bestimmt gemäß DIN ISO 22412:2008 (dynamische Lichtstreuung), im Bereich von 1 bis 500 nm. Ebenso betrifft die vorliegende Erfindung ein analytisches Hilfsmittel, herstellbar durch dieses Verfahren und eine Probennahmevorrichtung enthaltend das beschriebene zumindest teilweise beschichtete analytische Hilfsmittel.

### Stand der Technik

Im Stand der Technik werden eine Vielzahl analytischer Hilfsmittel, insbesondere zur schnellen und quantitativen analytischen Bestimmung von Bestandteilen flüssiger Proben, beispielsweise in Form von einzelnen Teststreifen oder bandförmigen Testmaterialien oder in integrierten Systemen, in denen das Testelement ein Teil einer Probennahmevorrichtung ist, beschrieben. Herkömmliche Testelemente werden aus Gründen der vereinfachten und kostengünstigeren Herstellung und der Bauteilstabilität in der Regel aus Kunststoff oder Metall gefertigt und weisen zumeist eine vergleichsweise hydrophobe Oberfläche auf, die für eine schnelle und gleichmäßige Benetzung des analytischen Hilfsmittels mit den flüssigen, zumeist wässrigen Proben nachteilig ist.

So sind beispielsweise aus WO 2007/045412 Probennahmevorrichtungen zur Entnahme von Körperflüssigkeiten bekannt, die ein Nadelelement aufweisen und die mit einem Testelement ausgestattet sind, mit dem zumindest ein Analyt in der Körperflüssigkeit qualitativ oder quantitativ erfasst werden kann. Der Transport der Körperflüssigkeitsprobe verläuft dabei entlang des Nadelelementes zum Testelement. Da in der Regel die Einstichstelle des Nadelelementes in die Haut von dem Testelement beabstandet ist, hat die Körperflüssigkeitsprobe längs der Kapillare in der Regel wenige Millimeter zu überwinden, um zu dem Testelement zu gelangen. Üblicherweise werden Nadelelemente aus Stahl verwendet, die eine relativ hydrophobe Stahloberfläche aufweisen, und demzufolge für den Transport der wässrigen Körperflüssigkeit weniger gut geeignet sind.

Zum Zweck der Erhöhung der Fließgeschwindigkeit der Körperflüssigkeit entlang des Nadelelementes beschreibt die EP 2 025 287 A1 die Modifizierung der Oberfläche des Nadelelementes mit einer hydrophilen Beschichtung. Als hydrophile Beschichtungen werden insbesondere nicht-ionische Tenside, wie Polysorbat, erwähnt.

In ähnlicher Weise beschreibt die EP 2 014 727 A1 hydrophilisierende metalloxidische Beschichtungen, wie zum Beispiel AlOOH, TiOx, SiO₂, oder dergleichen, wobei diese Beschichtungen beispielsweise in Form von diskreten Partikeln in Suspensionen aufgetragen und fixiert werden. Ebenfalls aus dem Stand der Technik bekannt ist die Verwendung von organischen polymeren Verbindungen, wie zum Beispiel PVP-PEG, von wasserlöslichen organischen Polysäuren beziehungsweise deren Salzen, wie zum Beispiel PAA oder Heparin-Salzen als hydrophile Beschichtung.

Zur Ausbildung einer hydrophilen Oberfläche des Nadelelements ist ferner eine Hydrophilisierung über physikalisch-chemische Maßnahmen möglich, wie zum Beispiel das Ätzen einer Stahloberfläche oder eine Plasma- oder Korona-Behandlung im Sinne der Schaffung aktiver Metalloberflächen. Üblicherweise sind diese Effekte vorübergehend und daher in der Regel nur Hilfsmaßnahmen zur Vorbereitung der Nadelelementoberfläche für eine nachfolgende Beschichtung.

Um den Transport der Körperflüssigkeit entlang des Nadelelementes zu verbessern, kann zudem eine kapillaraktive Oberflächenstruktur vorgesehen sein, wobei eine hydrophile Beschichtung eben dieser kapillaren Oberflächenstruktur besonders vorteilhaft für die Transportgeschwindigkeit der Körperflüssigkeit entlang des Nadelelementes sein kann.

In EP 1 887 355 A1 sind ein als Mikrofluidiksystem ausgebildetes Nadelelement zum kapillaren Transport einer Flüssigkeit sowie ein Verfahren zum Aufbringen einer hydrophilen Oberflächenbeschichtung auf ein derartiges Nadelelement beschrieben. Als Oberflächenbeschichtungsmaterialien werden Polyacrylsäure, Polyacrylat, Dextransulfat und/oder Chondroitinsulfat verwendet. Dabei wird besonders bevorzugt eine an dem Nadelelement als Mikrokanal ausgebildete Oberflächenstruktur mit einer derartigen hydrophilen Oberflächenbeschichtung versehen, um einen schnellen und sicheren Transport der Körperflüssigkeit entlang des Nadelelementes zu gewährleisten.

Die im Stand der Technik beschriebenen Beschichtungsmaterialien weisen jedoch oftmals eine unzureichende Stabilität auf, insbesondere eine unzureichende Langzeitstabilität. Als Gründe für eine unzureichende Stabilität sind beispielsweise eine zu geringe Bindung der Beschichtung an die beschichtete Oberfläche des Substrats zu nennen, die dazu führen kann, dass sich die Beschichtung während des Beschichtungsvorganges, oder bei der Lagerung oder bei der Verwendung des Substrats, zum Beispiel beim Einstechen eines auf diese Weise beschichteten Nadelelementes in die Haut, ablösen kann. Ebenso kann die hydrophile Beschichtung auch nicht beständig sein gegenüber längerer Einlagerung bei Raumtemperatur oder gegenüber Temperaturschwankungen. Des Weiteren zeigen manche Verbindungen eine geringe Stabilität, wenn sie Sterilisierungsbedingungen ausgesetzt werden und/oder eine geringe Beständigkeit gegenüber Ausgasungen aus einer Verpackung.

Insbesondere eine Instabilität gegenüber Verpackungsmaterialien führt häufig dazu, dass beispielsweise bei in Kunststoffmaterialien verpackten Substraten die Hydrophilie der beschichteten Oberflächen während der Lagerung deutlich abnimmt und eine ausreichende Hydrophilie der Substrate nach Lagerung nicht mehr gewährleistet ist. Dieser Verlust an Hydrophilie kann beispielsweise durch Adsorption von flüchtigen, in der Regel unpolaren, Bestandteilen der Verpackungsmaterialien erklärt werden.

Um einen solchen Verlust an Hydrophilie zu unterbinden, beschreibt die WO 2008/015227 die Verwendung spezifischer Verpackungen, die eine lose Abdeckung für die hydrophile Beschichtung und/oder die Zugabe von Adsorbentien in die Verpackung enthalten, um die Absorption von solchen flüchtigen Bestandteilen durch die hydrophile Beschichtung zu unterbinden. Die in WO 2008/015227 vorgeschlagenen Verpackungen sind jedoch kompliziert, kostspielig und insbesondere für ein automatisch funktionierendes Messgerät, welches auch das Auspacken der Substrate aus den Verpackungen zuverlässig bewerkstelligen muss, nachteilig.

In US 20070179373 A1 werden Vorrichtungen und Verfahren zur Aufnahme einer flüssigen Probe beschrieben. Unter anderem wird ein integriertes Aufnahmegerät offenbart, welches einen Analytdetektor und ein Gradientenmittel aufweist, um den Transport einer Probenflüssigkeit von einem Kontaktpunkt zu einem Detektionspunkt anzutreiben. Das integrierte Aufnahmegerät weist einen Schichtaufbau auf mit mehreren Schichten. Unter anderem ist eine erste Schicht und eine als Oberflächenschicht ausgebildete zweite Schicht vorgesehen. Eine hydrophile Oberfläche wird durch eine Pulverbeschichtung mit pyrogenen Silica-Nanopartikeln erzeugt.

Es besteht daher ein Bedarf an analytischen Hilfsmitteln mit hydrophilen Beschichtungen, die eine ausreichende Stabilität, insbesondere eine ausreichende Stabilität auch in Gegenwart von flüchtigen, in der Regel unpolaren, Bestandteilen von Verpackungsmaterialien, aufweisen.

### Aufgabe der Erfindung

Die vorliegende Erfindung beschäftigt sich demzufolge mit der Aufgabe, ein analytisches Hilfsmittel mit einer hydrophilen Beschichtung bereitzustellen, welche vorteilhafte Benetzungseigenschaften aufweist und sich durch eine hohe Stabilität der Beschichtung auszeichnet.

### Offenbarung der Erfindung

Erfindungsgemäß wird diese Aufgabe durch die Gegenstände der unabhängigen Ansprüche gelöst. Vorteilhafte Ausführungsformen sind Gegenstand der abhängigen Ansprüche.

Überraschenderweise wurde gefunden, dass bei Verwendung von Nanopartikeln mit Siliciumdioxidstruktur und einer mittleren Partikelgröße bestimmt gemäß DIN ISO 22412:2008 (dynamische Lichtstreuung) im Bereich von 1 bis 500 nm zur Beschichtung von analytischen Hilfsmitteln, analytische Hilfsmittel mit besonders vorteilhaften Eigenschaften bereitgestellt werden. Die so beschichteten analytischen Hilfsmittel zeichnen sich durch vorteilhafte Hydrophilie und Benetzbarkeit aus. Zudem ist eine hohe Stabilität der Beschichtung gegeben.

Demnach betrifft die vorliegende Erfindung auch ein analytisches Hilfsmittel, umfassend eine zumindest teilweise mit einer hydrophilen Beschichtung beschichtete Oberfläche, wobei die hydrophile Beschichtung Nanopartikel mit Siliciumdioxidstruktur und einer mittleren Partikelgröße bestimmt gemäß DIN ISO 22412:2008 (dynamische Lichtstreuung), im Bereich von 1 bis 500 nm enthält und die Nanopartikel Gruppen der Struktur (I) und/oder (II) umfassen 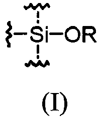 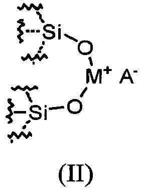 wobei in jeder der Gruppen der Struktur (I), unabhängig voneinander, R ausgewählt ist aus der Gruppe bestehend aus H, einem Metall enthaltenden Ion, 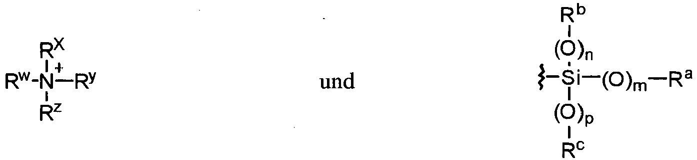 und wobei R^{w}, R^{x}, R^{y} und R^{z}, unabhängig voneinander, ausgewählt sind aus H und Alkyl,
und wobei R^{a}, R^{b} und R^{c}, unabhängig voneinander, optional substituierte Reste, ausgewählt aus der Gruppe bestehend aus H, Alkyl, Aryl, Heteroaryl, Cycloalkyl, Cycloheteroalkyl, Alkenyl und Alkoxyalkyl, sind,
und wobei n, m und p, unabhängig voneinander 0 oder 1 sind,
und wobei M⁺ ein Metallion und A- ein physiologisch verträgliches Anion ist.

Im Unterschied beispielsweise zur oben genannten US 20070179373 A1, welche lediglich eine hydrophile Oberflächenbeschichtung mit pyrogenen Silica-Nanopartikeln offenbart, umfassen die erfindungsgemäß vorgesehenen Nanopartikel also Gruppen der Struktur (I) und/oder (II).

Ebenso betrifft die vorliegende Erfindung ein Verfahren zur Herstellung eines analytischen Hilfsmittels, umfassend eine Oberfläche, die mindestens teilweise mit einer hydrophilen Beschichtung beschichtet ist, wobei das Verfahren umfasst:
(a) Bereitstellen des analytischen Hilfsmittels,
(b) Beschichten des analytischen Hilfsmittels durch Inkontaktbringen des analytischen Hilfsmittels mit einem Gemisch G umfassend mindestens ein Dispersionsmittel und Nanopartikel mit Siliciumdioxidstruktur, die eine mittlere Partikelgröße bestimmt gemäß DIN ISO 22412:2008, im Bereich von 1 bis 500 nm aufweisen,
   wobei die Nanopartikel Gruppen der Struktur (I) und/oder (II) umfassen 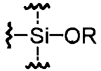 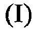 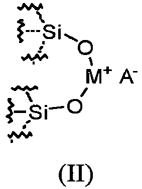 wobei in jeder der Gruppen der Struktur (I), unabhängig voneinander, R ausgewählt ist aus der Gruppe bestehend aus H, einem Metall enthaltenden Ion, 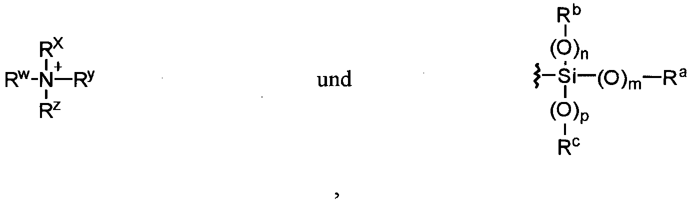 und wobei R^{w}, R^{x}, R^{y} und R^{z}, unabhängig voneinander, ausgewählt sind aus H und Alkyl,
   und wobei R^{a}, R^{b} und R^{c}, unabhängig voneinander, optional substituierte Reste, ausgewählt aus der Gruppe bestehend aus H, Alkyl, Aryl, Heteroaryl, Cycloalkyl, Cycloheteroalkyl, Alkenyl und Alkoxyalkyl, sind
   und wobei n, m und p, unabhängig voneinander 0 oder 1 sind,
   und wobei M⁺ ein Metallion und A⁻ ein physiologisch verträgliches Anion ist.
(c) Trocknen des gemäß (b) erhaltenen analytischen Hilfsmittels, und
(d) optional Sterilisieren des gemäß (c) erhaltenen analytischen Hilfsmittels,
wobei das Inkontaktbringen in (b) bevorzugt mittels Tauchbeschichtung und/oder Sprühbeschichtung und/oder Kontaktbeschichtung erfolgt, und wobei das analytische Hilfsmittel und/oder die Oberfläche des analytischen Hilfsmittels bevorzugt zumindest teilweise aus einem Metall und/oder einer Metalllegierung und/oder einem Metalloxid und/oder einem Mischmetalloxid und/oder einem Metallmischoxid besteht.

Gemäß einer weiteren Ausführungsform betrifft die vorliegende Erfindung auch die Verwendung von Nanopartikeln mit Siliciumdioxidstruktur und einer mittleren Partikelgröße bestimmt gemäß DIN ISO 22412:2008 (dynamische Lichtstreuung), im Bereich von 1 bis 500 nm als hydrophile Beschichtung für eine Oberfläche eines analytischen Hilfsmittels, bevorzugt als hydrophile Beschichtung für ein Nadelelement einer Probennahmevorrichtung zur Entnahme einer Körperflüssigkeit, wobei die Nanopartikel Gruppen der Struktur (I) und/oder (II) umfassen 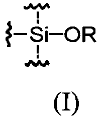 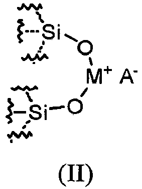 wobei in jeder der Gruppen der Struktur (I), unabhängig voneinander, R ausgewählt ist aus der Gruppe bestehend aus H, einem Metall enthaltenden Ion, 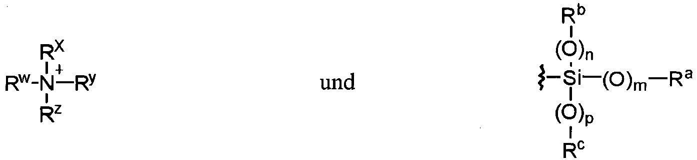 und wobei R^{w}, R^{x}, R^{y} und R^{z}, unabhängig voneinander, ausgewählt sind aus H und Alkyl, und wobei R^{a}, R^{b} und R^{c}, unabhängig voneinander, optional substituierte Reste, ausgewählt aus der Gruppe bestehend aus H, Alkyl, Aryl, Heteroaryl, Cycloalkyl, Cycloheteroalkyl, Alkenyl und Alkoxyalkyl, sind, und wobei n, m und p, unabhängig voneinander, 0 oder 1 sind, und wobei M⁺ ein Metallion und A- ein physiologisch verträgliches Anion ist.

### Nanopartikel

Wie oben bereits ausgeführt, umfasst die Beschichtung Nanopartikel mit Siliciumdioxidstruktur und einer mittleren Partikelgröße bestimmt gemäß DIN ISO 22412:2008 (dynamische Lichtstreuung), im Bereich von 1 bis 500 nm.

Bevorzugt weisen die Nanopartikel eine Größe im Bereich von 1 bis 100 nm, weiter bevorzugt im Bereich von 1 bis 50 nm, weiter bevorzugt im Bereich von 3 nm bis 25 nm, und besonders bevorzugt im Bereich von 5 bis 15 nm auf.

Die Nanopartikel weisen dabei bevorzugt eine BET-Oberfläche im Bereich von 100 bis 700 m²/g auf.

Die Beschichtung weist vorzugsweise eine mittlere Schichtdicke, welche im Folgenden auch als mittlere Dicke oder gemittelte Dicke bezeichnet wird, von höchstens 500 nm auf, beispielsweise von höchstens 300 nm. Die Schichtdicke kann nach üblichen Verfahren gemessen werden, beispielsweise mittels eines zerstörenden oder eines zerstörungsfreien Verfahrens. Insbesondere kann die Schichtdicke gemessen werden mittels eines Ellipsometers und/oder mittels eines Rasterkraftmikroskops und/oder mittels eines so genannten Alpha-Steppers, also einer Vorrichtung, welche eine Oberfläche der Schicht mittels einer Nadel abtastet und dabei die Auslenkung und/oder die Position der Nadel erfasst. Auch andere Messverfahren sind denkbar. Weiterhin kann, alternativ oder zusätzlich, die mittlere Schichtdicke auch rechnerisch und/oder semi-empirisch bestimmt werden. Beispielsweise kann die mittlere Schichtdicke aus einer bekannten Dichte der Beschichtung und einem Auftragsgewicht bestimmt werden. So können sich beispielsweise rechnerisch mittlere Schichtdicken im Bereich von 30 nm bis 300 nm, insbesondere im Bereich von 50 nm bis 150 nm, ergeben. Schwankungen können dabei unberücksichtigt bleiben, beispielsweise Schwankungen in einem oder mehreren Kanälen und/oder Kapillaren und/oder Schwankungen durch Randeffekte.

Was die Struktur der Nanopartikel betrifft, so handelt es sich, wie oben ausgeführt, um Nanopartikel mit Siliciumdioxidstruktur, die Gruppen der Struktur (I) und/oder (II) umfassen

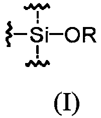

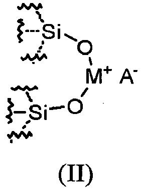

Bevorzugt ist zumindest ein Teil der Oberfläche der Nanopartikel mit Gruppen der Struktur (I) oder Gruppen der Struktur (II), bevorzugt mindestens mit Gruppen der Struktur (I), modifiziert.

Gemäß einer bevorzugten Ausführungsform betrifft die vorliegende Erfindung demnach ein analytisches Hilfsmittel, wie oben beschrieben, umfassend mindestens eine zumindest teilweise mit einer hydrophilen Beschichtung beschichtete Oberfläche, wobei die hydrophile Beschichtung Nanopartikel mit Siliciumdioxidstruktur, die Gruppen der Struktur (I) umfassen, enthält. Ebenso betrifft die vorliegende Erfindung ein Verfahren zur Herstellung eines analytischen Hilfsmittels, und ein analytisches Hilfsmittel herstellbar durch dieses Verfahren, wobei das analytische Hilfsmittel in Schritt (b) mit einem Gemisch G umfassend mindestens ein Dispersionsmittel und Nanopartikel mit Siliciumdioxidstruktur, wie oben beschrieben, beschichtet wird, wobei die Nanopartikel Gruppen der Struktur (I) aufweisen.

### Gruppen der Struktur (I)

Wie oben ausgeführt ist der Rest R ausgewählt aus der Gruppe bestehend aus H, einem Metall enthaltenden Ion, 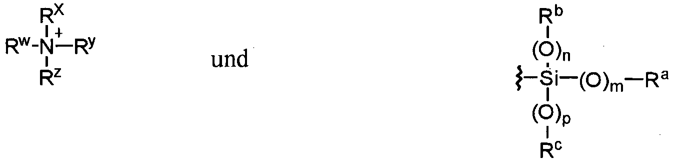 wobei R^{w}, R^{x}, R^{y} und R^{z}, unabhängig voneinander, ausgewählt sind aus H und Alkyl, und wobei R^{a}, R^{b} und R^{c}, unabhängig voneinander, optional substituierte Reste, ausgewählt aus der Gruppe bestehend aus H, Alkyl, Aryl, Heteroaryl, Cycloalkyl, Cycloheteroalkyl, Alkenyl und Alkoxyalkyl, sind, und wobei n, m und p, unabhängig voneinander, 0 oder 1 sind.

Wenn R H ist, umfassen die Siliciumdioxidpartikel demnach bevorzugt Gruppen der Struktur

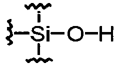

Demnach betrifft die vorliegende Erfindung auch ein analytisches Hilfsmittel, wie oben beschrieben, umfassend eine zumindest teilweise mit einer hydrophilen Beschichtung beschichtete Oberfläche, wobei die hydrophile Beschichtung Nanopartikel mit Siliciumdioxidstruktur, die Gruppen der Struktur 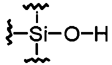 umfassen, enthält. Ebenso betrifft die vorliegende Erfindung ein Verfahren zur Herstellung eines analytischen Hilfsmittels, und ein analytisches Hilfsmittel herstellbar durch dieses Verfahren, wobei das analytische Hilfsmittel in Schritt (b) mit einem Gemisch G umfassend mindestens ein Dispersionsmittel und Nanopartikel mit Siliciumdioxidstruktur, wie oben beschrieben, beschichtet wird, wobei die Nanopartikel Gruppen der Struktur 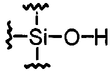 aufweisen.

Gemäß einer alternativen Ausführungsform ist R ein Metall enthaltendes Ion. In diesem Fall enthalten die Siliciumdioxidpartikel demnach bevorzugt Gruppen der Struktur

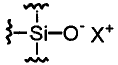

Gemäß einer bevorzugten Ausführungsform ist X⁺ ein Erdalkalimetall enthaltendes Ion, wie Mg²⁺ oder Ca²⁺, ein Alkalimetallion oder ein Aluminium enthaltendes Ion.

Wenn R ein Alkalimetallion ist, umfasst der Nanopartikel demnach bevorzugt Gruppen der Struktur 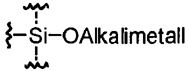 bzw. der Struktur:

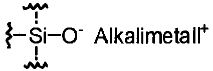

Besonders bevorzugt ist das Alkalimetall ausgewählt aus der Gruppe bestehend aus Li⁺, Na⁺, K⁺ und Mischungen davon. In diesem Zusammenhang bedeutet der Begriff "Mischungen davon", dass in jeder einzelnen Gruppe der Struktur

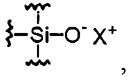

X⁺ unabhängig von jedem in jeder weiteren im Siliciumnanopartikel enthaltenen Gruppe der Struktur 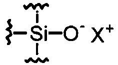 enthaltenem X⁺ ausgewählt ist aus der Gruppe bestehend aus Li⁺, Na⁺ und K⁺, und somit die Nanopartikel jeweils mehrere verschiedene Alkalimetallgegenionen aufweisen können. Ganz besonders bevorzugt ist X⁺ Na⁺.

Demnach betrifft die vorliegende Erfindung auch ein analytisches Hilfsmittel, wie oben beschrieben, umfassend eine zumindest teilweise mit einer hydrophilen Beschichtung beschichtete Oberfläche, wobei die hydrophile Beschichtung Nanopartikel mit Siliciumdioxidstruktur, die Gruppen der Struktur 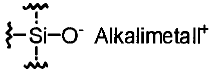 umfassen, enthält. Ebenso betrifft die vorliegende Erfindung ein Verfahren zur Herstellung eines analytischen Hilfsmittels und ein analytisches Hilfsmittel herstellbar durch dieses Verfahren, wobei das analytische Hilfsmittel in Schritt (b) mit einem Gemisch G umfassend mindestens ein Dispersionsmittel und Nanopartikel mit Siliciumdioxidstruktur, wie oben beschrieben, beschichtet wird, wobei die Nanopartikel Gruppen der Struktur 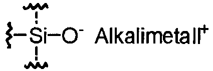 aufweisen.

Gemäß einer alternativen Ausführungsform ist R ein Kation der folgenden Struktur 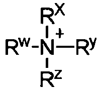 wobei R^{w}, R^{x}, R^{y} und R^{z}, unabhängig voneinander, ausgewählt sind aus H und Alkyl.

Unter dem Begriff "Alkyl", wie er im Rahmen der vorliegenden Erfindung verwendet wird, werden unverzweigte oder verzweigte, optional substituierte Alkylreste verstanden.

Ist R^{w} und/oder R^{x} und/oder R^{y} und/oder R^{z} ein Alkylrest, so handelt es sich bevorzugt um einen Alkylrest ausgewählt aus der Gruppe bestehend aus Methyl, Ethyl, Propyl, isoPropyl, Butyl, sec-Butyl und tert.-Butyl, bevorzugt Methyl.

Ist R 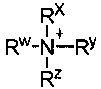 so sind R^{w}, R^{x}, R^{y} und R^{z} besonders bevorzugt, unabhängig voneinander, ausgewählt aus H, Methyl und Ethyl. Besonders bevorzugt sind R^{w}, R^{x}, R^{y} und R^{z} H.

Demnach betrifft die vorliegende Erfindung auch ein analytisches Hilfsmittel, wie oben beschrieben, umfassend eine zumindest teilweise mit einer hydrophilen Beschichtung beschichtete Oberfläche, wobei die hydrophile Beschichtung Nanopartikel mit Siliciumdioxidstruktur, die Gruppen der Struktur 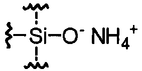 umfassen, enthält. Ebenso betrifft die vorliegende Erfindung ein Verfahren zur Herstellung eines analytischen Hilfsmittels, und ein analytisches Hilfsmittel herstellbar durch dieses Verfahren, wobei das analytische Hilfsmittel in Schritt (b) mit einem Gemisch G umfassend mindestens ein Dispersionsmittel und Nanopartikel mit Siliciumdioxidstruktur, wie oben beschrieben, beschichtet wird, wobei die Nanopartikel Gruppen der Struktur 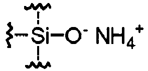 aufweisen.

Solche Nanopartikel sind beispielsweise unter dem Namen Ludox^{®} AS-30 (Grace, Aldrich) oder Levasil^{®} 200N/30%. (EKA) erhältlich.

Gemäß einer besonders bevorzugten Ausführungsform der vorliegenden Erfindung ist R 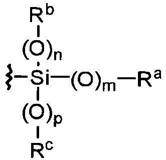 und wobei R^{a}, R^{b} und R^{c}, unabhängig voneinander, optional substituierte Reste, ausgewählt aus der Gruppe bestehend aus H, Alkyl, Aryl, Heteroaryl, Cycloalkyl, Cycloheteroaklyl, Alkenyl und Alkoxyalkyl, sind, und wobei n, m und p, unabhängig voneinander, 0 oder 1 sind,

Gemäß einer bevorzugten Ausführungsform der Erfindung ist R 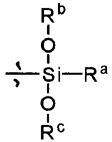 wobei p und n 1 sind und m 0 ist.

Demnach betrifft die vorliegende Erfindung auch ein analytisches Hilfsmittel wie oben beschrieben, umfassend eine zumindest teilweise mit einer hydrophilen Beschichtung beschichtete Oberfläche, wobei die hydrophile Beschichtung Nanopartikel mit Siliciumdioxidstruktur, die Silan-modifizierte Siliciumgruppen der Struktur 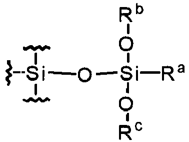 umfassen, enthält. Ebenso betrifft die vorliegende Erfindung ein Verfahren zur Herstellung eines analytischen Hilfsmittels, und ein analytisches Hilfsmittel herstellbar durch dieses Verfahren, wobei das analytische Hilfsmittel in Schritt (b) mit einem Gemisch G umfassend mindestens ein Dispersionsmittel und Nanopartikel mit Siliciumdioxidstruktur, wie oben beschrieben, beschichtet wird, wobei die Nanopartikel Silan-modifizierte Siliciumgruppen der Struktur 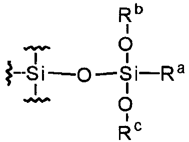 aufweisen.

Enthalten die Siliciumnanopartikel Silan-modifizierte Siliciumgruppen der Struktur 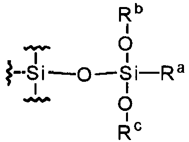 so können die Siliciumnanopartikel gegebenenfalls weitere Silangruppen-Modifikation enthalten, beispielsweise Gruppen der Struktur 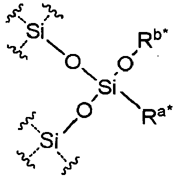 und/oder Gruppen der Struktur 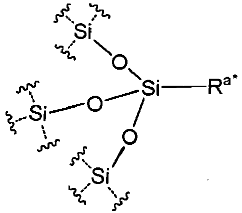 und/oder Gruppen der Struktur 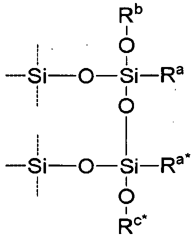 und/oder ähnliche verzweigte Silangruppen-Modifikationen,
in denen R^{a*}, R^{b*} und R^{c*}, unabhängig voneinander, optional substituierte Reste, ausgewählt aus der Gruppe bestehend aus H, Alkyl, Aryl, Heteroaryl, Cycloalkyl, Cycloheteroaklyl, Alkenyl und Alkoxyalkyl sind.

Unter dem Begriff "substituierter Alkylrest", wie er im Rahmen der vorliegenden Erfindung verwendet wird, sind Alkylreste zu verstehen, in denen mindestens ein H durch einen geeigneten Substituenten ersetzt wurde. Ein substituierter Alkylrest kann dabei mindestens einen, beispielsweise 2, 3, 4, 5, 6, 7, 8, 9, 10, 11 oder 12 Substituenten enthalten, wobei, falls mehr als ein Substituent vorhanden ist, die vorhandenen Substituenten gleich oder voneinander verschieden sein können. Was die Art der Substituenten betrifft, so bestehen im Prinzip keine Einschränkungen, vorausgesetzt, dass bei Verwendung der Nanopartikel eine Beschichtung mit ausreichender Hydrophilie und Stabilität bereitgestellt werden kann. Die Substituenten können beispielsweise ausgewählt sein aus der Gruppe bestehend aus Epoxy, Aryl, Alkenyl, Alkinyl, Halogen, Hydroxyl, Alkylcarbonyloxy, Arylcarbonyloxy, Alkoxycarbonyloxy, Aryloxycarbonyloxy, Carboxylat, Alkylcarbonyl, Arylcarbonyl, Alkoxycarbonyl, Aminocarbonyl, Alkylaminocarbonyl, Dialkylaminocarbonyl, Alkylthiocarbonyl, Alkoxy, Phosphat, Phosphonat, Phosphinat, Phosphorsäureester, Amino, Acylamino, Alkylcarbonylamino, Arylcarbonylamino, Carbamate, Carbamide, Amidine, Nitro, Imino, SH, Alkylthio, Arylthio, Thiocarboxylat, Sulfat, Alkylsulfinyl, Sulfonat, Sulfamoyl, Sulfonamido, Trifluoromethyl, Cyano, Azido, Aldehyd, Ketogruppe, Cycloalkyl wie z.B. Cyclopentyl oder Cyclohexyl, Heterocycloalkyl wie z.B. Morpholino, Piperazinyl oder Piperidinyl, und Gycosyl. Besonders bevorzugte Substituenten sind Hydroxylgruppen, Glycosylgruppen und Phosphorsäureestergruppen.

Unter dem Begriff "Cycloalkyl" sind im Rahmen der Erfindung optional substituierte, cyclische Alkylreste zu verstehen, wobei es sich um monocyclische oder polycyclische Gruppen handeln kann. Als bevorzugtes Beispiel für einen Cycloalkylrest sei optional substituiertes Cylohexyl genannt.

Unter dem Begriff "Cycloheteroalkyl", wie er im Rahmen der vorliegenden Erfindung verwendet wird, sind optional substituierte, cyclische Alkylreste zu verstehen, die mindestens ein Heteroatom, wie O, N oder S im Ring aufweisen, wobei es sich um monocyclische oder polycyclische Gruppen handeln kann.

Unter den Begriffen "substituierter Cycloalkylrest" oder "Cycloheteroalkyl", wie sie im Rahmen der vorliegenden Erfindung verwendet werden, sind Cycloalkylreste bzw. Cycloheteroalkylreste zu verstehen, in denen mindestens ein H durch einen geeigneten Substituenten ersetzt wurde. Ein substituierter Cycloalkylrest bzw. Cycloheteroalkylrest kann dabei mindestens einen, beispielsweise 2, 3, 4, 5, 6, 7, 8, 9, 10, 11 oder 12 Substituenten enthalten, wobei, falls mehr als ein Substituent vorhanden ist, die vorhandenen Substituenten gleich oder voneinander verschieden sein können. Was die Art der Substituenten betrifft, so wird auf die im Hinblick auf substituierte Alkylreste beispielhaft genannten Substituenten verwiesen.

Unter dem Begriff "Aryl", wie er im Rahmen der vorliegenden Erfindung verwendet wird, werden optional substituierte, 5- und 6-gliedrige aromatische Ringe, als auch substituierte oder unsubstituierte polycyclische aromatische Gruppen (Arylgruppen) verstanden, beispielsweise tricyclische oder bicyclische Arylgruppen. Beispielhaft seien, optional substituierte, Phenylgruppen oder Naphtylgruppen genannt. Polycyclische aromatische Gruppen können dabei auch nicht aromatische Ringe enthalten.

Unter dem Begriff "Heteroaryl", wie im Rahmen der vorliegenden Erfindung verwendet, werden, optional substituierte, 5- und 6-gliedrige aromatische Ringe, als auch substituierte oder unsubstituierte polycyclische aromatische Gruppen verstanden, beispielsweise tricyclische oder bicyclische Arylgruppen, enthaltend ein oder mehrere, beispielsweise 1 bis 4, wie 1, 2, 3, oder 4, Heteroatome im Ringsystem. Wenn mehr als ein Heteroatom im Ringsystem vorhanden ist, so können die vorhandenen mindestens zwei Heteroatome gleich oder verschieden sein. Beispielhaft seien die folgenden Heteroaryl-Reste genannt: Benzodioxolyl, Pyrrolyl, Furanyl, Thiophenyl, Thiazolyl, Isothiazolyl, Imidazolyl, Triazolyl, Tetrazolyl, Pyrazolyl, Oxazolyl, Isoxazolyl, Pyridinyl, Pyrazinyl, Pyridazinyl, Benzoxazolyl, Benzodioxazolyl, Benzothiazolyl, Benzoimidazolyl, Benzothiophenyl, Methylenedioxyphenylyl, Napthyridinyl, Chinolinyl, Isochinolinyl, Indolyl, Benzofuranyl, Purinyl, Benzofuranyl, Deazapurinyl oder Indolizinyl.

Unter dem Begriff "substituierter Arylrest oder Heteroarylrest", wie er im Rahmen der vorliegenden Erfindung verwendet wird, sind Arylreste oder Heteroarylreste zu verstehen, in denen mindestens ein H durch einen geeigneten Substituenten ersetzt wurde. Ein substituierter Arylrest oder Heteroarylrest kann dabei mindestens einen, beispielsweise 2, 3, 4, 5, 6, 7, 8, 9, 10, 11 oder 12 Substituenten enthalten, wobei, falls mehr als ein Substituent vorhanden ist, die vorhandenen Substituenten gleich oder voneinander verschieden sein können. Was die Art der Substituenten betrifft, so bestehen im Prinzip keine Einschränkungen, vorausgesetzt, dass die Verbindung M eine ausreichende Hydrophilie aufweist und/oder mit der Verbindung M eine ausreichend stabile Beschichtung bereitgestellt werden kann. Die Substituenten können beispielsweise ausgewählt sein aus der Gruppe bestehend aus Aryl, Alkenyl, Alkynyl, Halogen, Hydroxyl, Alkylcarbonyloxy, Arylcarbonyloxy, Alkoxycarbonyloxy, Aryloxycarbonyloxy, Carboxylat, Alkylcarbonyl, Arylcarbonyl, Alkoxycarbonyl, Aminocarbonyl, Alkylaminocarbonyl, Dialkylaminocarbonyl, Alkylthiocarbonyl, Alkoxy, Phosphat, Phosphonat, Phosphinat, Phosphorsäureester, Amino, Acylamino, Alkylcarbonylamino, Arylcarbonylamino, Carbamate, Carbamide, Amidine, Nitro, Imino, SH, Alkylthio, Arylthio, Thiocarboxylat, Sulfat, Alkylsulfinyl, Sulfonat, Sulfamoyl, Sulfonamido, Trifluoromethyl, Cyano, Azido, Cycloalkyl wie z.B. Cyclopentyl oder Cyclohexyl, Heterocycloalkyl wie z.B. Morpholino, Piperazinyl oder Piperidinyl, Zuckerreste und Heteroaryl.

Unter dem Begriff "Alkoxyalkyl" werden Alkylreste verstanden, die ein oder mehrere Gruppen -O- innerhalb der Alkylkette umfassen. Der Begriff umfasst Gruppen der Struktur Alkyl-O-Alkyl und ähnliche Strukturen, wobei die jeweiligen Alklyreste, wie oben beschrieben, substituiert oder unsubstituiert sein können.

Unter dem Begriff Alkenyl sind Alkylreste zu verstehen, die mindestens eine C-C-Doppelbindung aufweisen. Was die Art möglicher Substituenten betrifft, wird auf obige Ausführungen verwiesen.

Gemäß einer bevorzugten Ausführungsform der Erfindung sind R^{a}, R^{b} und R^{c}, unabhängig voneinander, ausgewählt aus der Gruppe bestehend aus H, optional substituierten Alkylresten und optional substituierten Alkyoxyalkylresten.

Demnach betrifft die vorliegende Erfindung auch ein analytisches Hilfsmittel, wie oben beschrieben, umfassend eine zumindest teilweise mit einer hydrophilen Beschichtung beschichtete Oberfläche, wobei die hydrophile Beschichtung Nanopartikel mit Siliciumdioxidstruktur, die Gruppen der Struktur (I), umfasst, wobei R 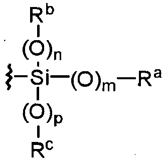 ist, und wobei R^{a}, R^{b} und R^{c}, unabhängig voneinander, ausgewählt sind aus der Gruppe bestehend aus H, optional substituierten Alkylresten und optional substituierten Alkyoxyalkylresten.

Ebenso betrifft die vorliegende Erfindung ein Verfahren zur Herstellung eines analytischen Hilfsmittels, und ein analytisches Hilfsmittel herstellbar durch dieses Verfahren, wobei das analytische Hilfsmittel in Schritt (b) mit einem Gemisch G umfassend mindestens ein Dispersionsmittel und Nanopartikel mit Siliciumdioxidstruktur, wie oben beschrieben, beschichtet wird, wobei die Nanopartikel Gruppen der Struktur (I) umfassen, wobei R 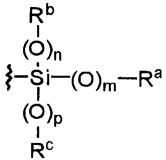 ist, und wobei R^{a}, R^{b} und R^{c}, unabhängig voneinander, ausgewählt sind aus der Gruppe bestehend aus H, optional substituierten Alkylresten und optional substituierten Alkyloxyalkylresten.

Beispielhaft seien die folgenden bevorzugten Gruppen für die Reste R^{a}, R^{b} bzw. R^{c}, genannt:

### Beispiele für mit Gruppen der Struktur

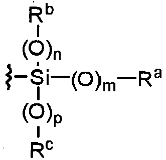

modifizierte Siliziumdioxidnanopartikel sind solche, die beispielsweise mit den folgenden Silanen modifiziert sind: Octyltriethoxysilan, Methyltrimethoxysilan, Methyltriethoxysilan, Methyltriisopropoxysilan, Isocyanatsilan, Beta-(3,4-epoxycyclohexyl)-ethyl-trimethoxysilan; Epoxy-Silane oder Silane, die eine Glycidoxy- und/oder eine Glycidoxypropylgruppe aufweisen, wie Gamma-Glycidoxypropyltrimethoxysilan, Gamma-Glycidoxypropyltriethoxysilan, Gammaglycidoxypropylmethyldiethoxysilan, 3-glycidoxypropyl-hexyltrimethoxysilan, Beta-(3,4-epoxycyclohexyl)-ethyltriethoxysilan, Silane, die eine Vinylgruppe enthalten, wie Vinyltriethoxysilan, Anhydridsilane, also Silane, die eine cyclische organische Anhydrid-Einheit tragen wie z.B. Succinanhydrid oder Maleinsäureanhydrid, und deren Hydrolyseprodukte, und/oder Aminosilane wie 3-Aminopropyltri(m)ethoxysilan und Di-Triaminosilane.

Besonders bevorzugt sind R^{a}, R^{b} und R^{c}, unabhängig voneinander, ausgewählt aus der Gruppe bestehend aus H, Alkyl und Gruppen der folgenden Formeln: 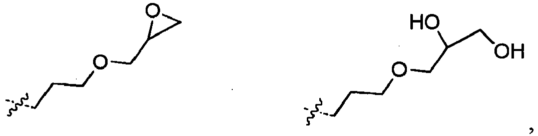 wobei die Alkylgruppe weiter bevorzugt Methyl oder Ethyl, besonders bevorzugt Ethyl ist.

Ganz besonders bevorzugt sind p und n 1 und m = 0 und R^{b} und R^{c} sind Methyl oder Ethyl, bevorzugt, Ethyl, und R^{c} ist weiter bevorzugt

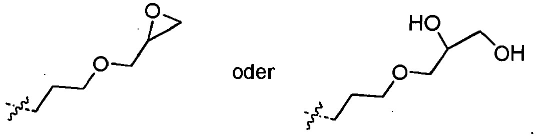

Demnach betrifft die vorliegende Erfindung auch ein analytisches Hilfsmittel, wie oben beschrieben, umfassend eine zumindest teilweise mit einer hydrophilen Beschichtung beschichtete Oberfläche, wobei die hydrophile Beschichtung Nanopartikel mit Siliciumdioxidstruktur enthält, wobei die Gruppe der Formel (I) eine Struktur der Formel (Ia) oder (Ib) aufweist:

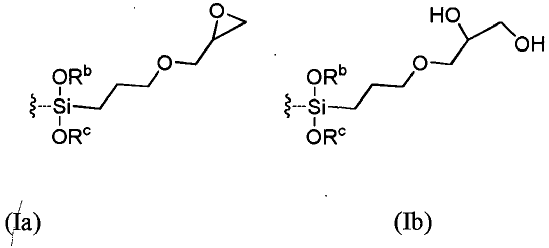

Ebenso betrifft die vorliegende Erfindung ein Verfahren zur Herstellung eines analytischen Hilfsmittels, und ein analytisches Hilfsmittel herstellbar durch dieses Verfahren, wobei das analytische Hilfsmittel in Schritt (b) mit einem Gemisch G umfassend mindestens ein Dispersionsmittel und Nanopartikel mit Siliciumdioxidstruktur, wie oben beschrieben, beschichtet wird, wobei die Gruppe der Formel (I) eine Struktur der Formel (Ia) oder (Ib) aufweist: 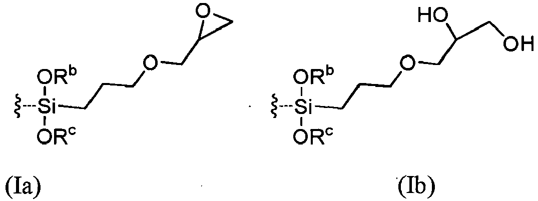 und wobei R^{b} und R^{c}, unabhängig voneinander, ausgewählt sind aus der Gruppe bestehend aus H und Alkyl, bevorzugt H, Methyl und Ethyl.

Solche Nanopartikel sind beispielsweise unter dem Namen Bindzil^{®} CC30 bzw. CC301 und CC40 bzw. CC401 (eka Akzo Nobel) erhältlich.

Enthalten die Nanopartikel Gruppen der Formel (I), in denen R 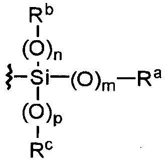 ist, so haben bevorzugt 10 bis 50 % aller Gruppen R der im Siliciumnanopartikel befindlichen Gruppen 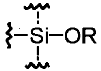 die Struktur der Formel

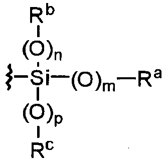

Die verbleibenden 50 % bis 90 % der im Nanopartikel enthaltenden Gruppen R sind H und/oder ein Metall enthaltendes Ion und/oder 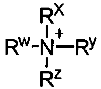 je nachdem, mit welchem Gegenion die Nanopartikel stabilisiert sind. Bevorzugt sind die verbleibenden Gruppen R, wie oben beschrieben, H und/oder Na⁺.

Demnach betrifft die vorliegende Erfindung auch ein analytisches Hilfsmittel, wie oben beschrieben, umfassend eine zumindest teilweise mit einer hydrophilen Beschichtung beschichtete Oberfläche, wobei die hydrophile Beschichtung Nanopartikel mit Siliciumdioxidstruktur, die Gruppen der Struktur 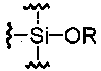 umfassen, enthält, wobei bevorzugt 5 bis 50 % aller Gruppen R eine Struktur der Formel 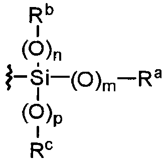 aufweisen. Ebenso betrifft die vorliegende Erfindung ein Verfahren zur Herstellung eines analytischen Hilfsmittels, und ein analytisches Hilfsmittel herstellbar durch dieses Verfahren, wobei das analytische Hilfsmittel in Schritt (b) mit einem Gemisch G umfassend mindestens ein Dispersionsmittel und Nanopartikel mit Siliciumdioxidstruktur, wie oben beschrieben, beschichtet wird, wobei die Nanopartikel Gruppen der Struktur 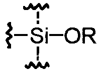 aufweisen, und wobei bevorzugt 5 bis 50 % aller Gruppen R eine Struktur der Formel 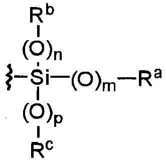 aufweisen.

### Gruppen der Struktur (II)

Gemäß einer weiteren Ausführungsform der Erfindung weisen die Siliziumdioxid-Nanopartikel zusätzlich oder alternativ zu den Gruppen der Formel (I), Gruppen der Formel (II) 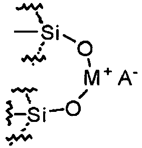 auf. Insbesondere weisen die Nanopartikel entweder Gruppen der Formel (I) oder Gruppen der Formel (II) auf, d.h. wenn die Nanopartikel Gruppen der Formel (I) aufweisen, weisen die bevorzugt keine Gruppen der Formel (II) auf und umgekehrt.

M ist ein Metall, bevorzugt ein trivalentes Metall, weiter bevorzugt Aluminium. Besonders bevorzugt ist M demnach Aluminium bzw. M⁺ = Al⁺.

Demnach betrifft die vorliegende Erfindung auch ein analytisches Hilfsmittel, wie oben beschrieben, umfassend eine zumindest teilweise mit einer hydrophilen Beschichtung beschichtete Oberfläche, wobei die hydrophile Beschichtung Nanopartikel mit Siliciumdioxidstruktur enthält und die Nanopartikel Gruppen der Struktur (IIa) 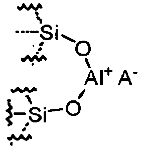 umfassen. Ebenso betrifft die vorliegende Erfindung ein Verfahren zur Herstellung eines analytischen Hilfsmittels, und ein analytisches Hilfsmittel herstellbar durch dieses Verfahren, wobei das analytische Hilfsmittel in Schritt (b) mit einem Gemisch G umfassend mindestens ein Dispersionsmittel und Nanopartikel mit Siliciumdioxidstruktur, wie oben beschrieben, beschichtet wird, wobei die Nanopartikel Gruppen der Struktur (IIa) 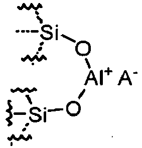 umfassen.

Was die chemische Natur des Anions A⁻ angeht, so bestehen diesbezüglich keinerlei Einschränkungen, vorausgesetzt, das Anion ist physiologisch verträglich und verfälscht bei Verwendung des analytischen Hilfsmittels das Messergebnis nicht. Bevorzugte Anionen sind beispielsweise Chlorid, Bromid, Iodid, Sulfat, Hydrogensulfat, Phosphat und Hydrogenphosphat. Besonders bevorzugt ist A⁻ Chlorid.

Demnach betrifft die vorliegende Erfindung auch ein analytisches Hilfsmittel, wie oben beschrieben, umfassend eine zumindest teilweise mit einer hydrophilen Beschichtung beschichtete Oberfläche, wobei die hydrophile Beschichtung Nanopartikel mit Siliciumdioxidstruktur enthält und die Nanopartikel Gruppen der Struktur (IIa) 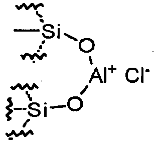 umfassen. Ebenso betrifft die vorliegende Erfindung ein Verfahren zur Herstellung eines analytischen Hilfsmittels, und ein analytisches Hilfsmittel herstellbar durch dieses Verfahren, wobei das analytische Hilfsmittel in Schritt (b) mit einem Gemisch G umfassend mindestens ein Dispersionsmittel und Nanopartikel mit Siliciumdioxidstruktur, wie oben beschrieben, beschichtet wird, wobei die Nanopartikel Gruppen der Struktur (IIa) 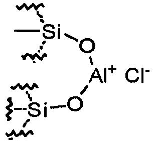 umfassen.

Nanopartikel enthaltende Gruppen der Struktur (IIa) werden beispielswiese durch Behandeln von Nanopartikeln mit Aluminiumchlorid erhalten. Solche Nanopartikel sind beispielswiese als Bindzil^{®} CAT erhältlich (Akzo Nobel).

### Siliciumdioxidstruktur

Die Nanopartikel weisen Siliciumdioxidstruktur auf, wobei es sich bevorzugt um amorphes Siliciumdioxid handelt. Weiterhin können die Nanopartikel neben Si und O und den Gruppen R bzw. M⁺ weitere Fremdatome aufweisen. Solche Fremdatome können beispielsweise Sn, Ti, Zr, Hf, Ge, In, Ga, P, Al oder B sein. Falls vorhanden, ersetzen die Fremdatome dabei ein oder mehrere Si Atome in der Siliciumdioxidstruktur.

Gemäß einer Ausführungsform der Erfindung enthalten die Nanopartikel Aluminium als Fremdion.

Demnach betrifft die vorliegende Erfindung auch ein analytisches Hilfsmittel, wie oben beschrieben, umfassend eine zumindest teilweise mit einer hydrophilen Beschichtung beschichtete Oberfläche, wobei die hydrophile Beschichtung Nanopartikel mit Siliciumdioxidstruktur enthält, wobei die Siliciumdioxidstruktur der Nanopartikel mindestens ein Fremdatom, ausgewählt aus der Gruppe bestehend aus Sn, Ti, Zr, Hf, Ge, In, Ga, P, Al und B, aufweist, bevorzugt wobei das Fremdatom Al ist.

Ebenso betrifft die vorliegende Erfindung ein Verfahren zur Herstellung eines analytischen Hilfsmittels, und ein analytisches Hilfsmittel herstellbar durch dieses Verfahren, wobei das analytische Hilfsmittel in Schritt (b) mit einem Gemisch G umfassend mindestens ein Dispersionsmittel und Nanopartikel mit Siliciumdioxidstruktur, wie oben beschrieben, beschichtet wird, wobei die Siliciumdioxidstruktur der Nanopartikel mindestens ein Fremdatom, ausgewählt aus der Gruppe bestehend aus Sn, Ti, Zr, Hf, Ge, In, Ga, P, Al und B, aufweist, bevorzugt wobei das Fremdatom Al ist.

Enthält die Siliciumdioxidstruktur der Nanopartikel mindestens Aluminium, so enthalten die Nanopartikel Aluminum bevorzugt in einer Menge im Bereich von 0,1 bis 10 Gew.%, bezogen auf das Gesamtgewicht des Nanopartikels.

### Verfahren zur Herstellung des analytischen Hilfsmittels

Was das Beschichten des analytischen Hilfsmittels durch Inkontaktbringen des analytischen Hilfsmittels mit dem Gemisch G in Schritt (b) des oben beschriebenen Verfahrens betrifft, so kann dieses Beschichten auf alle dem Fachmann bekannten Methoden durchgeführt werden. Bevorzugt erfolgt das Inkontaktbringen mittels Tauchbeschichtung und/oder Sprühbeschichtung und/oder Kontaktbeschichtung. Alternativ oder zusätzlich kommen jedoch zahlreiche andere Verfahren in Betracht. So kann beispielsweise ein Verfahren angewandt werden, ausgewählt aus: einer Tauchbeschichtung, einer Sprühbeschichtung, einem Aufschleudern (Spin Coating), einem Druckverfahren, einem Rakelverfahren oder einem Tropfverfahren. Auch Kombinationen der genannten Verfahren und/oder anderer Verfahren sind jedoch grundsätzlich einsetzbar.

### Gemisch G

Was das in Schritt (b) verwendete Gemisch betrifft, so enthält G, wie oben beschrieben, neben Nanopartikeln mindestens ein Dispersionsmittel. Die Nanopartikel liegen bevorzugt dispergiert bzw. kolloidal in dem mindestens einen Dispersionsmittel gelöst vor.

Bevorzugt handelt es sich bei dem mindestens einen Dispersionsmittel um Wasser.

Gemisch G kann weiterhin mindestens ein Lösungsmittel enthalten, beispielswiese mindestens ein organisches Lösungsmittel, bevorzugt ein polares organisches Lösungsmittel, weiter bevorzugt ein polares, protisches organisches Lösungsmittel, weiter bevorzugt ein Alkohol. Gemäß einer bevorzugten Ausführungsform ist das mindestens eine organische Lösungsmittel mit Wasser mischbar. Besonders bevorzugt ist das mindestens eine organische Lösungsmittel, ein Lösungsmittel ausgewählt aus der Gruppe bestehend aus Methanol, Ethanol, 1-Propanol, 2-Propanol, Methoxymethanol, Methoxyethanol und Ethylenglykol. Enthält G mindestens ein organisches Lösungsmittel, so enthält das Lösungsmittelgemisch bevorzugt höchstens 50 Vol.-% des organischen Lösungsmittels, weiter bevorzugt in einer Menge im Bereich von 0,1 Vol.-% bis 30 Vol.-%, weiter bevorzugt im Bereich von 1 Vol.-% bis 20 Vol.-%, und besonders bevorzugt im Bereich von 1,5 Vol.-% bis 5 Vol.-%, bezogen auf das Gesamtvolumen des Gemisches G.

Demnach betrifft die vorliegende Erfindung auch ein Verfahren zur Herstellung, wie oben beschrieben, und ein analytisches Hilfsmittel, herstellbar oder hergestellt durch dieses Verfahren, wobei das Gemisch G weiterhin mindestens ein Lösungsmittel in einer Menge von höchstens 50 Vol.-% enthält.

Was die Menge an Nanopartikeln in Gemisch G betrifft, so enthält Gemisch G die Nanopartikel bevorzugt in einer Menge im Bereich von 0,01 Gew.% bis 5 Gew.%, bevorzugt in einer Menge im Bereich von 0,02 Gew.% bis 3 Gew.%, weiter bevorzugt von 0,05 Gew.% bis 2 Gew.%, bezogen auf das Gesamtgewicht des Gemisches G.

Demnach betrifft die vorliegende Erfindung auch ein Verfahren zur Herstellung eines analytischen Hilfsmittels, wie oben beschrieben, und ein analytisches Hilfsmittel, herstellbar oder hergestellt durch dieses Verfahren, wobei das Gemisch G die Nanopartikel bevorzugt in einer Menge im Bereich von 0,01 Gew.% bis 5 Gew.%, bezogen auf das Gesamtgewicht des Gemisches G enthält.

Bevorzugt besteht Gemisch G im Wesentlichen aus dem mindestens einen Dispersionsmittel und den Nanopartikeln mit Siliciumdioxidstruktur. "Im Wesentlichen" bedeutet, dass das Gemisch weitere Bestandteile, wie Verunreinigungen, in einer Menge von höchstens 1 Gew.-%, bevorzugt in einer Menge von höchstens 0,1 Gew.-%, aufweist.

Erfolgt die Beschichtung in Schritt (b) durch Tauchbeschichtung, wie oben beschrieben, so ist die Verweildauer des analytischen Hilfsmittels eingetaucht in Gemisch G bevorzugt maximal 5 Minuten, weiter bevorzugt maximal 2 Minuten, weiter bevorzugt maximal 1 Minute, beispielsweise im Bereich von 1 sec bis 30 sec.

Das oben beschriebene Verfahren zur Herstellung des analytischen Hilfsmittels umfasst neben den oben beschriebenen Schritten weiterhin (c) das Trocknen des gemäß (b) erhaltenen analytischen Hilfsmittels.

Trocknen bedeutet im Rahmen der Erfindung, dass auf dem analytischen Hilfsmittel befindliches Dispersionsmittel und gegebenenfalls weiteres vorhandenes Lösungsmittel im Wesentlichen vollständig, bevorzugt vollständig entfernt wird. Dabei kann die Trocknung in einem Trockenschrank bei einer vorbestimmten Temperatur von bevorzugt 20 bis 150 Grad Celsius, besonders bevorzugt von 20 bis 120 Grad Celsius und ganz besonders bevorzugt von 20 bis 80 Grad Celsius vorgenommen werden. Zusätzlich oder alternativ dazu kann das Trocknen durch Beströmung des analytischen Hilfsmittels mit einem Gas, beispielsweise Luft erfolgen.

Alternativ oder zusätzlich dazu kann das Trocknen auch durch Lagern des beschichteten analytischen Hilfsmittels an der Luft oder in einer inerten Atmosphäre bei einer Temperatur im Bereich von 20 bis 150 Grad Celsius, bevorzugt bei einer Temperatur im Bereich von 20 bis 80 Grad Celsius, weiter bevorzugt bei Raumtemperatur, erfolgen.

Schritt (c) kann gemäß einer Ausführungsform der Erfindung auch einen zusätzlichen Waschschritt enthalten. In diesem Fall wird das gemäß (b) erhaltene analytische Hilfsmittel zunächst getrocknet, wie oben beschrieben, das analytische Hilfsmittel anschließend mit einem Lösungsmittel oder Lösungsmittelgemisch gewaschen, bevorzugt mit Wasser, wobei das Waschen bevorzugt für eine Zeit von maximal 1 Minute, beispielsweise im Bereich von 1 sec bis 30 sec, durchgeführt wird. Im Anschluss an diesen optionalen Waschschritt wird das analytische Hilfsmittel bevorzugt nochmals getrocknet, wobei im zweiten Trocknungsschritt das auf dem analytischen Hilfsmittel befindliche Lösungsmittel oder Lösungsmittelgemisch, welches zum Waschen verwendet wurde, im Wesentlichen vollständig, bevorzugt vollständig entfernt wird. Die Trocknungsschritte können dabei auf gleiche Weise oder verschieden durchgeführt werden. Bevorzugt wird in (c) kein Waschschritt durchgeführt.

Das gemäß (c) erhaltene analytische Hilfsmittel, wird bevorzugt in einem weiteren Schritt (d) sterilisiert.

Das Sterilisieren erfolgt dabei bevorzugt durch Bestrahlung mit Gammastrahlung und/oder Betastrahlung. Weitere, alternativ oder zusätzlich einsetzbare Möglichkeiten sind eine Dampfsterilisation und/oder ein Autoklavieren und/oder eine chemische Sterilisation, beispielsweise mit Ethylenoxid (ETO). Allgemein kann beispielsweise mindestens ein Sterilisierungsverfahren eingesetzt werden, ausgewählt aus der Gruppe bestehend aus: einer Strahlensterilisation, insbesondere mit Gammastrahlung und/oder Betastrahlung, einer thermischen Sterilisation, insbesondere einer trockenen thermischen Sterilisation und/oder einer Dampfsterilisation, einer chemischen Sterilisation mit mindestens einem keimabtötenden Medium, insbesondere einem keimabtötenden gasförmigen und/oder flüssigen Medium.

Beispielsweise können die erfindungsgemäßen analytischen Hilfsmittel in geeigneten Behältnissen wie beispielsweise Kunststoffbeuteln verpackt werden, wobei das Verpacken beispielsweise durch Einschweißen erfolgen kann. Die Sterilisation gemäß (d) erfolgt insbesondere nach Verpacken oder Einschweißen der analytischen Hilfsmittel, insbesondere durch Bestrahlung mit Gammastrahlung.

Besonders bevorzugt wird das analytische Hilfsmittel vor Schritt (b) mit mindestens einem Ätzmittel und/oder Plasma behandelt.

Demnach betrifft die vorliegende Erfindung auch ein Verfahren, wie oben beschrieben, und ein analytisches Hilfsmittel herstellbar oder hergestellt durch dieses Verfahren, wobei die zu beschichtende Oberfläche des analytischen Hilfsmittels vor Schritt (b) mit mindestens einem Ätzmittel und/oder mit Plasma behandelt wird.

Gemäß einer Ausführungsform kann das analytische Hilfsmittel vor, beispielsweise kurz vor Schritt (b), mit einem Ätzmittel geätzt werden. Unter "kurz vor" soll dabei im Rahmen der vorliegenden Erfindung eine Zeitspanne verstanden werden, welche vorzugsweise 12 Stunden, insbesondere 8 Stunden, besonders bevorzugt 1 Stunde und insbesondere 10 Minuten nicht überschreitet. So wird in Schritt (b) beispielsweise ein frisch geätztes analytisches Hilfsmittel eingesetzt. Unter einem frisch geätzten analytischen Hilfsmittel ist ein analytisches Hilfsmittel zu verstehen, das mit einem Ätzmittel kurz vor der Behandlung mit Gemisch G oberflächlich angeätzt worden ist. Es ist frisch geätzt, wenn zwischen dem Ätzen des analytischen Hilfsmittels und der Behandlung des analytischen Hilfsmittels mit Gemisch G bevorzugt maximal 0 bis 12 Stunden, besonders bevorzugt 0 bis 8 Stunden und ganz besonders bevorzugt 0 bis 4 Stunden, insbesondere sogar 0 bis 1 Stunde oder sogar nicht mehr als 10 Minuten verstreichen. Werden die jeweiligen Zeitgrenzen eingehalten, so liegt ein frisch geätztes analytisches Hilfsmittel im Sinne der vorliegenden Erfindung vor.

Das gegebenenfalls geätzte und anschließend gegebenenfalls mit Wasser gewaschene analytische Hilfsmittel wird in dieser Zwischenzeit, zwischen Ätzen und Beschichten, bevorzugt beispielsweise in gegebenenfalls mit einem Stabilisierungsmittel versehenem Wasser aufbewahrt. Gegebenenfalls wird das analytische Hilfsmittel vor Schritt (b) getrocknet. Was die Trocknungsbedingungen betrifft, so wird auf die obigen Ausführungen verwiesen. Das Ätzen erfolgt dabei bevorzugt mit einem Ätzmittel, welches Salpetersäure oder eine Eisen(III)chlorid-Lösung und Salzsäure umfasst.

Wird Salpetersäure verwendet, so wird bevorzugt eine 20- bis 40-Gew.%ige Salpetersäure verwendet, besonders bevorzugt eine 25- bis 35-Gew.%ige und ganz besonders bevorzugt eine 30- bis 34-Gew.%ige Salpetersäure.

Gemäß einer weiteren Ausführungsform wird die zu beschichtende Oberfläche, gegebenenfalls die wie oben beschrieben geätzte Oberfläche, des analytischen Hilfsmittels kurz vor Schritt (b) mit Plasma behandelt. So wird in Schritt (b) bevorzugt ein frisch mit Plasma behandeltes analytisches Hilfsmittel eingesetzt. Unter einem frisch mit Plasma behandelten analytischen Hilfsmittel ist ein analytisches Hilfsmittel zu verstehen, das mit Plasma kurz vor der Behandlung mit Gemisch G behandelt ist. Es ist frisch mit Plasma behandelt, wenn zwischen dem Behandeln des analytischen Hilfsmittels mit Plasma und der Behandlung des analytischen Hilfsmittels mit Gemisch G bevorzugt maximal 0 bis 12 Stunden, besonders bevorzugt 0 bis 8 Stunden und ganz besonders bevorzugt 0 bis 4 Stunden, insbesondere sogar 0 bis 1 Stunde oder sogar nicht mehr als 10 Minuten verstreichen.

### Hydrophile Beschichtung

Der Begriff "hydrophile Beschichtung", wie er im Rahmen der vorliegenden Erfindung verwendet wird, bedeutet eine Beschichtung, deren Oberfläche im Vergleich zur nicht beschichteten Oberfläche des analytisches Hilfsmittels einen kleineren, bevorzugt um mindestens 15° kleineren, Kontaktwinkel mit Wasser aufweist, gemessen vorzugsweise gemäß DIN 55660.2. Auch eine Messung gemäß DIN EN 828:1997 wäre grundsätzlich geeignet. Bevorzugt weist die Oberfläche der Beschichtung einen Kontaktwinkel mit Wasser von kleiner 60° auf, bevorzugt von kleiner 50°, weiter bevorzugt von kleiner 40°, und insbesondere bevorzugt von kleiner 30°, gemessen vorzugsweise gemäß DIN 55660.2, wobei grundsätzlich wiederum auch DIN EN 828:1997 geeignet wäre.

Was die Menge der Nanopartikel auf der Oberfläche des analytischen Hilfsmittels betrifft, so ist das analytische Hilfsmittel bevorzugt mit einer Menge an Nanopartikeln im Bereich von 7,5 µg pro mm² Oberfläche des analytischen Hilfsmittels bis 150 ng pro mm² Oberfläche des analytischen Hilfsmittels beschichtet.

Bevorzugt bestehen mindestens 90 Gew.-%, weiter bevorzugt mindestens 95 Gew.-%, weiter bevorzugt mindestens 96 Gew.-%, weiter bevorzugt mindestens 97 Gew.-%, weiter bevorzugt mindestens 98 Gew.-%, weiter bevorzugt mindestens 99 Gew.-%, weiter bevorzugt mindestens 99,5 Gew.-%, weiter bevorzugt mindestens 99,9 Gew.-%, weiter bevorzugt 100 Gew.-%, der hydrophilen Beschichtung aus Nanopartikeln mit Siliciumdioxidstruktur.

Im Rahmen der Erfindung können die Beschichtungen dabei Mischungen verschiedener Nanopartikel enthalten. Bevorzugt enthalten die Beschichtungen nur Nanopartikel einer Struktur.

Im Rahmen der Erfindung kann die Oberfläche des analytischen Hilfsmittels teilweise oder vollständig mit der hydrophilen Beschichtung beschichtet sein. Bevorzugt ist die Oberfläche, die bei Verwendung des analytischen Hilfsmittels der Probe zugewandt ist, zumindest teilweise oder vollständig beschichtet. Bevorzugt sind mindestens 80 %, weiter bevorzugt mindestens 90 %, weiter bevorzugt mindestens 95 %, weiter bevorzugt mindestens 98 %, und besonders bevorzugt mindestens 99 % der Oberfläche beschichtet.

Handelt es sich bei dem analytischen Hilfsmittel beispielsweise um ein Nadelelement, beispielsweise mit mindestens einem Innenraum, insbesondere mindestens einer Kanüle und/oder mindestens einer Kapillare, so wird bevorzugt die gesamte Oberfläche des Innenraums des Nadelelementes hydrophil beschichtet und besonders bevorzugt mindestens eine kapillaraktive Oberflächenstruktur von einer Spitze des Nadelelementes bis zu einer optionalen Kontaktstelle des Nadelelementes mit mindestens einem optionalen Testelement, beispielsweise mit mindestens einer Testchemie zum Nachweis mindestens eines Analyten in einer Körperflüssigkeit.

### Oberfläche des analytischen Hilfsmittels

Was die chemische Natur des analytischen Hilfsmittels betrifft, besteht dessen Oberfläche bevorzugt zumindest teilweise aus einem Metall und/oder einer Metalllegierung und/oder einem Metalloxid und/oder einem Metalloxidgemisch. Dies bedeutet, dass das analytische Hilfsmittel an sich aus einem Metall und/oder einer Metalllegierung und/oder einem Metalloxid und/oder einem Metalloxidgemisch besteht oder dass das analytische Hilfsmittel zumindest teilweise aus einem anderen Material hergestellt ist und dieses Material zumindest teilweise mit einem Metall und/oder einer Metalllegierung und/oder einem Metalloxid und/oder einem Metalloxidgemisch beschichtet wurde.

Gemäß einer Ausführungsform umfasst das analytische Hilfsmittel demnach eine Beschichtung aus Metall und/oder einer Metalllegierung und/oder einem Metalloxid und/oder einem Mischmetalloxid und/oder einem Metallmischoxid.

Die Beschichtung aus Metall und/oder einer Metalllegierung und/oder einem Metalloxid und/oder einem Mischmetalloxid und/oder einem Metallmischoxid kann in jeglicher bekannter Weise aufgebracht werden, beispielsweise durch Sputtern, Metallbedampfung, galvanische Beschichtung oder Abscheiden aus gelösten Metallverbindungen. Ferner können auch mehrere Schichten aus Metall und/oder einer Metalllegierung und/oder einem Metalloxid und/oder einem Mischmetalloxid und/oder einem Metallmischoxid aufgetragen sein.

Gemäß einer Ausführungsform der Erfindung kann das analytische Hilfsmittel, beispielsweise im Fall, dass das analytische Hilfsmittel, wie unten beschrieben, ein Testelement oder Verteilerelement ist, mittels Metallbedampfung, beispielsweise mit Aluminium, beschichtet werden. Das so erhaltene analytische Hilfsmittel wird anschließend beispielsweise durch Oxidation zu einem Metalloxid und/oder einem Metallmischoxid, insbesondere zu Böhmit, umgewandelt. Die Oxidation erfolgt beispielsweise mit Wasser, Alkali- oder Erdalkalihydroxiden, Sauerstoff, Wasserstoffperoxid, Ozon, Wärme in Anwesenheit von Luftsauerstoff oder Schwefelverbindungen. Die metallische Oberfläche wird dabei zumindest teilweise aufoxidiert, beispielsweise durch das Böhmit-Verfahren mit heißem Wasser und/oder Wasserdampf.

Wird eine solche Beschichtung aufgebracht, so werden diese Metall- und/oder Metalllegierung- und/oder dieses Metalloxid- und/oder Mischmetalloxid- und/oder diese Metallmischoxid-enthaltende Beschichtung vorzugsweise vor Schritt (b) auf dem analytischen Hilfsmittel aufgebracht. Demnach beschreibt die vorliegende Erfindung auch ein Verfahren zur Herstellung eines analytischen Hilfsmittels, umfassend eine zumindest teilweise mit einer hydrophilen Beschichtung beschichtete Oberfläche, und ein analytisches Hilfsmittel hergestellt oder herstellbar durch dieses Verfahren, wobei Schritt (a) des oben beschriebenen Verfahrens umfasst:
(a) Bereitstellen des analytischen Hilfsmittels, wobei das Bereitstellen das Beschichten des analytischen Hilfsmittels mit einem Metall und/oder einer Metalllegierung und/oder einem Metalloxid und/oder einem Mischmetalloxid und/oder einem Metallmischoxid umfasst,

Handelt es sich bei dem analytischen Hilfsmittel um ein Nadelelement, wird das analytische Hilfsmittel bevorzugt nicht mit einem Metall und/oder einer Metalllegierung und/oder einem Metalloxid und/oder einem Mischmetalloxid und/oder einem Metallmischoxid beschichtet.

Im Falle, dass die Nanopartikel Gruppen der Struktur (I) umfassen 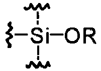 in denen R ein Metall enthaltendes Ion, oder 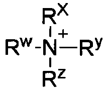 ist, werden die Nanopartikel gemäß einer bevorzugten Ausführungsform der Erfindung vor Schritt (b) de-ionisiert. Bei diesem De-ionisieren werden die Nanopartikel, enthaltend die Gruppen der Strukturen 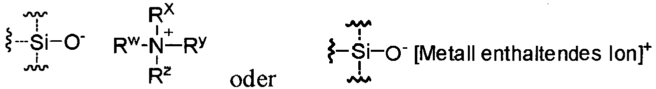 in Nanopartikel mit Gruppen der Struktur 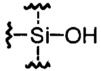 umgewandelt.

Das De-ionisieren kann dabei generell nach allen dem Fachmann bekannten Verfahren erfolgen. Bevorzugt erfolgt das De-ionisieren durch Ionenaustauschchromatographie bzw. durch Inkontaktbringen mit einem sauren Ionentauschmaterial.

Demnach betrifft die vorliegende Erfindung ein Verfahren zur Herstellung eines analytischen Hilfsmittels, und ein analytisches Hilfsmittel herstellbar durch dieses Verfahren,
(a) Bereitstellen des analytischen Hilfsmittels,
(a1) Bereitstellen von Nanopartikeln mit Siliciumdioxidstruktur, die eine mittlere Partikelgröße bestimmt gemäß DIN ISO 22412:2008, im Bereich von 1 bis 500 nm aufweisen, wobei die Nanopartikel Gruppen 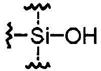 aufweisen, durch De-ionisieren von Nanopartikeln mit Siliciumdioxidstruktur, wobei die Nanopartikel Gruppen der Struktur 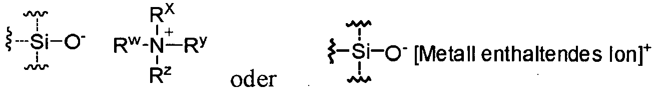 aufweisen, wobei R^{w}, R^{x}, R^{y} und R^{z}, unabhängig voneinander, ausgewählt sind aus H und Alkyl,
(b) Beschichten des analytischen Hilfsmittels durch Inkontaktbringen des analytischen Hilfsmittels mit einem Gemisch G umfassend mindestens ein Dispersionsmittel und die gemäß (a1) erhaltenen Nanopartikel
(c) Trocknen des gemäß (b) erhaltenen analytischen Hilfsmittels, und
(d) optional Sterilisieren des gemäß (c) erhaltenen analytischen Hilfsmittels,
wobei das Inkontaktbringen in (b) bevorzugt mittels Tauchbeschichtung und/oder Sprühbeschichtung und/oder Kontaktbeschichtung und/oder mindestens einem anderen der oben genannten Verfahren erfolgt, und wobei das analytische Hilfsmittel und/oder die Oberfläche des analytischen Hilfsmittels bevorzugt zumindest teilweise aus einem Metall und/oder einer Metalllegierung und/oder einem Metalloxid und/oder einem Mischmetalloxid und/oder einem Metallmischoxid besteht.

Demnach betrifft die vorliegende Erfindung bevorzugt auch ein analytisches Hilfsmittel, wie oben beschrieben, umfassend eine zumindest teilweise mit einer hydrophilen Beschichtung beschichtete Oberfläche, wobei die hydrophile Beschichtung Nanopartikel mit Siliciumdioxidstruktur enthält, wobei die Nanopartikel Gruppen der Struktur 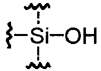 enthält.

### Analytisches Hilfsmittel:

Unter dem Begriff "analytisches Hilfsmittel", wie im Rahmen der vorliegenden Erfindung verwendet, ist allgemein ein Element zu verstehen, mittels dessen oder mittels dessen Hilfe mindestens eine Eigenschaft mindestens einer Probe bestimmt werden kann. Grundsätzlich kommen diesbezüglich beliebige analytische Hilfsmittel in Betracht, beispielsweise starre analytische Hilfsmittel oder auch verformbare analytische Hilfsmittel. Das analytische Hilfsmittel kann insbesondere ganz oder teilweise aus einem Werkstoff bestehen, ausgewählt aus der Gruppe bestehend aus einem Metall, einem Metalloxid, einem Papier, einer Keramik, einem Kunststoff und einem Textilwerkstoff wie beispielsweise Cellulose oder Kunstfasern. Auch Kombinationen der genannten Materialien und/oder anderer Materialien sind denkbar.

Insbesondere kann das analytische Hilfsmittel ein analytisches Hilfsmittel zur qualitativen und/oder quantitativen Analyse von Proben sein.

Bei der Probe kann es sich insbesondere um eine flüssige oder gasförmige Probe handeln. Besonders bevorzugt umfasst die Probe mindestens eine Körperflüssigkeit oder Teile davon, insbesondere Blut oder Blutbestandteile, interstitielle Flüssigkeit, Speichel oder Urin.

Bei der mindestens einen Eigenschaft kann es sich grundsätzlich um eine beliebige chemische, physikalische oder biologische Eigenschaft handeln. Besonders bevorzugt umfasst die mindestens eine Eigenschaft eine Konzentration mindestens eines Analyten in der Probe, insbesondere mindestens eines Metaboliten, wie beispielsweise Glucose, Laktat oder Cholesterin.

Besonders bevorzugt ist das analytische Hilfsmittel somit ein analytisches Hilfsmittel, welches im Rahmen eines Nachweises mindestens eines Analyten in einer Flüssigkeit, insbesondere einer Körperflüssigkeit, einsetzbar ist. Insbesondere kann das analytische Hilfsmittel ein analytisches Hilfsmittel sein, welches bei einer Probennahme, beispielsweise bei einer Entnahme einer Probe einer Körperflüssigkeit, und/oder bei einer Probenanalyse der Probe, beispielsweise bei einem Nachweis mindestens eines Analyten in einer Körperflüssigkeit, eingesetzt werden kann. Insbesondere kann das analytische Hilfsmittel demnach ein analytisches Hilfsmittel oder ein Teil eines analytischen Hilfsmittels zur qualitativen und/oder quantitativen Analyse von Blut und/oder Interstitialflüssigkeit sein.

Besonders bevorzugt eignet sich das analytische Hilfsmittel zum Vermessen und/oder zum qualitativen und/oder quantitativen Nachweis von mindestens einem Analyten der Körperflüssigkeit, beispielsweise des Blutes und/oder der Interstitialflüssigkeit, beispielsweise von Zucker, bevorzugt Glucose, Lipiden, Stoffwechselprodukten, wie z. B. Harnstoff bzw. Harnsäure, Proteinen, Peptiden und Salzen, oder andere Inhaltsstoffe des Blutes oder der Interstitialflüssigkeit. Bei mindestens einem nachzuweisenden Analyten kann es sich beispielsweise um mindestens einen Metaboliten handeln, beispielsweise um Blutglucose, Laktat, Cholesterin oder andere Metabolite.

Was die Art des analytischen Hilfsmittels betrifft, so sind alle dem Fachmann bekannten analytischen Hilfsmittel umfasst. Bevorzugt ist das analytische Hilfsmittel ausgewählt aus der Gruppe bestehend aus einem Nadelelement, einem Testelement zum Nachweis mindestens eines Analyten in einer Flüssigkeit, und optional einem Verteilerelement zur Verteilung einer aufgenommenen Probe einer Körperflüssigkeit.

### Testelement

Unter "Testelement" werden im Rahmen der vorliegenden Erfindung alle trägergebundenen Elemente zum Nachweis mindestens eines Analyten in einer Flüssigkeit verstanden. Ein Testelement kann dabei prinzipiell zum Nachweis mindestens eines Analyten für medizinische oder nichtmedizinische Zwecke hergerichtet sein. Diese trägergebundenen Testelemente umfassen vorzugsweise eine oder mehrere Schichten, enthaltend mindestens ein Nachweisreagenz zum Nachweis des jeweils interessierenden mindestens einen Analyten. Bevorzugt sind diese Nachweisreagenzien in entsprechenden Schichten eines Trägers eingebettet. Ein Inkontaktbringen des Testelementes und insbesondere des Nachweisreagenz mit der Flüssigkeit führt bei Anwesenheit des mindestens einen nachzuweisenden Analyten, welcher auch als Zielanalyt bezeichnet werden kann, zu einer nachweisbaren Veränderung, beispielsweise physikalischer und/oder chemischer Art, beispielsweise unter Ausbildung kovalenter, nicht-kovalenter oder Komplexbindungen zwischen Nachweisreagenz und Zielanalyt. Dies führt vorzugsweise zu einem messbaren Signal, beispielsweise einem elektrischen Signal und/oder einem Farbumschlag, bevorzugt zu einem messbaren optischen Signal, welches beispielsweise visuell oder mit Hilfe eines Geräts beispielsweise reflexionsphotometrisch oder fluoreszenzphotometrisch ausgewertet werden kann.

Was die äußere Erscheinungsform des Testelements betrifft, so sind alle dem Fachmann geläufigen Erscheinungsformen im Rahmen der Erfindung umfasst. Insbesondere kann das Testelement ein Teststreifen, ein Testband oder eine Testscheibe sein. Im Folgenden werden, ohne Beschränkung weiterer, alternativ oder zusätzlich einsetzbarer Ausführungsformen, insbesondere flache, streifenförmige Testelemente, also Teststreifen, beschrieben.

Zusätzlich zu dem mindestens einen Nachweisereagenz, welches mindestens eine Analytspezifische Reaktion durchführen kann, kann das Testelement, insbesondere mindestens ein Testfeld des Testelements, weitere Substanzen enthalten, beispielsweise Trägerstoffe, Hilfsstoffe, Pigmente, Füllstoffe, Pufferstoffe oder Ähnliches. Zwischen weiteren Stoffen, die ebenfalls an der Reaktion zum Nachweis des Analyten beteiligt sind, und dem eigentlichen Nachweisreagenz, wird im Folgenden dabei nicht unterschieden. Insbesondere kann das Nachweisreagenz ein enzymatisches Nachweisreagenz umfassen. Als Beispiele derartiger Glucose-spezifischer enzymatischer Nachweisreagenzien sind Deoxireductase-Enzyme (z.B. GlucDOR/PQQ), Dehydrogenase-Enzyme, Oxidase-Enzyme oder ähnliche Enzyme zu nennen, beispielsweise Glucose- Oxidase (GOD) oder Glucosedehydrogenase.

Bei der mindestens einen nachweisbaren Reaktion handelt es sich, wie oben bereits ausgeführt, bevorzugt um eine optisch nachweisbare Reaktion. Auch andere Arten von Reaktionen sind jedoch grundsätzlich möglich. Insbesondere kann es sich um eine Reaktion handeln, bei welcher bei Anwesenheit des mindestens einen Zielanalyten mindestens ein Nachweisstoff gebildet wird. Dabei können auch mehrere Nachweisstoffe gebildet und/oder verwendet werden, die einzeln, in Gruppen oder alle nachgewiesen werden können. Nachweisstoffe sind also Stoffe, die sich aufgrund der mindestens einen Nachweisreaktion bilden und/oder die an der mindestens einen Nachweisreaktion beteiligt sind und die nachweisbar sind. Anhand des nachgewiesenen mindestens einen Nachweisstoffs kann beispielsweise der mindestens eine Analyt quantitativ und/oder qualitativ nachgewiesen werden. Es sind aber auch Nachweise und/oder Nachweisstoffe möglich, bei denen der Nachweis des mindestens einen Nachweisstoffs nicht oder nicht nur zum Nachweis des Analyten verwendet wird, sondern alternativ beispielsweise zur Bestimmung der Volumenschicht der Probendicke oberhalb des Testfelds, wie im Folgenden noch beschrieben wird.

Bevorzugt ist die oben beschriebene Testchemie zum Nachweis mindestens eines Zielanalyten mit mindestens einem Nachweisreagenz unempfindlich gegenüber der hydrophilen Beschichtung. Dies gewährleistet, dass, sollten sich Spuren der hydrophilen Beschichtung bei Verwendung des analytischen Hilfsmittels ablösen, das Testelement kontaminiert werden kann ohne die Testchemie zu beeinflussen. Somit wird aufgrund der Unempfindlichkeit der Testchemie gegenüber der Beschichtung durch Spuren der Verbindung der Formel (I) oder Abbauprodukte derselben der Test zum Nachweis des Zielanalyten nicht oder nur unwesentlich verfälscht. Bevorzugt löst sich bei der bestimmungsgemäßen Benutzung im Wesentlichen kein, weiter bevorzugt kein, Anteil der hydrophilen Beschichtung vom analytischen Hilfsmittel.

Was den Aufbau des Testelements betrifft, so weist dieses bevorzugt mindestens ein Trägerelement auf, insbesondere einen Trägerstreifen. Dieses Trägerelement kann beispielsweise ein Kunststoffmaterial, ein Keramikmaterial, ein Papiermaterial, ein Kompositmaterial oder Ähnliches umfassen. Das Testfeld kann dann auf dieses Trägerelement aufgebracht sein. Das Trägerelement kann eingesetzt werden, um eine mechanisch tragende Funktion für das Testelement zu übernehmen, beispielsweise um eine Halterung des Testelements während des Aufbringens der Probe und/oder während einer Messung zu ermöglichen. Die mindestens eine hydrophile Beschichtung kann beispielsweise ganz oder teilweise auf das Trägerelement und/oder insbesondere auf die Testchemie und/oder auf ein oder mehrere andere Elemente des Testelements aufgebracht sein

Das Trägerelement kann beispielsweise mindestens ein Folienelement umfassen, insbesondere mindestens eine Kunststofffolie. Das Folienelement weist vorzugsweise eine geschlossene Oberfläche auf, welche vorzugsweise im Wesentlichen undurchlässig ist gegenüber der Probe. Vorzugsweise ist das Folienelement nicht-porös ausgestaltet, weist eine nicht-poröse Oberfläche auf oder weist Poren mit einem mittleren Porenradius von nicht mehr als 5 µm, vorzugsweise von nicht mehr als 1 µm, auf. Damit steht das Folienelement beispielsweise im Gegensatz zu herkömmlichen netzartigen Materialien, beispielsweise Spreitnetzen. Allgemein können das Trägerelement und/oder die Trägeroberfläche mindestens ein für die Probe im Wesentlichen undurchlässiges Material aufweisen.

### Verteilerelement

Unter einem Verteilerelement ist im Rahmen der Erfindung eine Vorrichtung zu verstehen, mit der eine Verteilung einer aufgenommenen Probe, insbesondere einer flüssigen Probe, über einen zweidimensionalen Verteilungsbereich und/oder ein dreidimensionales Verteilungsvolumen erreicht werden kann. Insbesondere kann das Verteilerelement zu einer gleichmäßigen, flächigen und schnellen Verteilung der zu analysierenden Probe, bevorzugt kleiner Blutproben, bevorzugt auf Testelementen, beispielsweise Teststreifen, insbesondere Glucoseteststreifen, eingerichtet sein.

Das Verteilerelement kann insbesondere ausgewählt sein aus der Gruppe bestehend aus einer Kapillarstruktur, einer Membran, einem Netz, insbesondere einem Spreitnetz. Bevorzugt handelt es sich bei dem Verteilerelement im Rahmen der Erfindung um ein Spreitnetz. Unter einer Membran ist im Rahmen der vorliegenden Erfindung ein poröses Element zu verstehen, beispielsweise eine poröse Folie oder poröse Schicht, welche eingerichtet ist, um die Probe aufzunehmen und vorzugsweise lateral, also parallel zu einer Schichtebene der Folie bzw. Schicht, zu verteilen. Insbesondere kann die Membran mindestens eine poröse Kunststofffolie umfassen. Der Begriff "Spreitnetz" wird im Rahmen der vorliegenden Erfindung ausdrücklich als Gattungsbegriff für alle zu Spreit- bzw. Verteil- oder Transferzwecken geeignete Filamentstrukturen verstanden. Darin eingeschlossen sind u.a. Gewebe, Gewirke, Gestricke und Vliese. Der Begriff "Filament" umfasst sowohl Mono- als auch Poly-Filamente einheitlicher oder nicht-einheitlicher Materialbasis und Dimensionierung. Es ist zu erwähnen, dass der Probentransfer vorzugsweise durch die Filamentstruktur hindurch erfolgt.

Gemäß einer bevorzugten Ausführungsform liegen die Membran und/oder das Spreitnetz auf mindestens einer Schicht eines Testelements auf, beispielsweise einer Nachweisschicht des Testelements, enthaltend das mindestens eine Nachweisreagenz, und/oder einer Abtrennschicht, enthaltend mindestens ein reflektierendes Pigment und/oder mindestens eine Substanz, welche zum Abtrennen von Probenbestandteilen, beispielsweise roten Blutkörperchen, eingerichtet ist. Die aufgetragene Probenflüssigkeit kann also beispielsweise von dem erfindungsgemäß vorgesehenen Spreitnetz über Kapillarwirkungen zur Schicht enthaltend das mindestens eine Nachweisreagenz geleitet und an den Kontaktstellen von Spreitnetz und Nachweisschicht ebenfalls durch Kapillarkräfte auf der Nachweisschicht gespreitet bzw. verteilt werden. Das Spreitnetz dient somit vorzugsweise als Hilfsmittel für ungerichtete (isotrope) flächige Verteilung einer Flüssigprobe am Zielort, nämlich auf der mindestens einen Schicht enthaltend das mindestens eine Nachweisreagenz. Dabei tritt nur im Zusammenspiel mit dem Spreitnetz die gewünschte Zwischenspeicherung und Flächenausbreitung der Probe auf, indem das Spreitnetz gegenüber der Nachweisschicht eine Vielzahl variierender kapillaraktiver Zwischenräume bzw. Kapillarspalte begrenzt, die aufgrund der Oberflächenkontur der Filamente und deren räumlicher Anordnung in ihrer Gesamtheit weitgehend ungerichtet verlaufen.

Wie oben beschrieben umfasst die Oberfläche des analytischen Hilfsmittels bevorzugt ein Metall und/oder eine Metalllegierung und/oder ein Metalloxid und/oder ein Mischmetalloxid. Demnach ist das analytische Hilfsmittel, gemäß einer bevorzugten Ausführungsform, ein Verteilerelement umfassend eine Oberfläche aus einem Metall und/oder einer Metalllegierung und/oder einem Metalloxid und/oder einem Mischmetalloxid. Bevorzugt ist das analytische Hilfsmittel im Rahmen der Erfindung ein Verteilerelement, beispielsweise ein Spreitnetz, welches mit einem Metall und/oder einer Metalllegierung und/oder einem Metalloxid und/oder einem Mischmetalloxid beschichtet sein kann, wie oben beschrieben.

Demnach betrifft die vorliegende Erfindung auch ein analytisches Hilfsmittel, wobei das analytische Hilfsmittel ein gegebenenfalls mit einem Metall und/oder einer Metalllegierung und/oder einem Metalloxid und/oder einem Mischmetalloxid beschichtetes Spreitnetz ist.

### Nadelelement

Gemäß einer alternativen bevorzugten Ausführungsform ist das analytische Hilfsmittel ein Nadelelement oder umfasst ein derartiges Nadelelement. Dabei kann ein Nadelelement grundsätzlich ein beliebiges Element mit Spitze oder Schneide sein, welches zum Erzeugen einer Öffnung in einer Hautpartie eines Benutzers eingerichtet ist. So können als Nadelelement z.B. eine Lanzette, eine Kanüle, insbesondere eine Hohlkanüle, eine zumindest teilweise offene Hohlkanüle, ein Micromesser oder dergleichen, eine Kapillare, insbesondere ein Kapillarspalt, eingesetzt werden.

Handelt es sich bei dem Nadelelement um eine Lanzette, so kann die Lanzette insbesondere einen so genannten Microsampler umfassen, also ein Element, welches sowohl eine Spitze und/oder Schneide zum Erzeugen eines Einstichs als auch mindestens einen Kapillarkanal zur Aufnahme der Probe aufweist. Weiterhin kann der Microsampler optional mindestens ein Testelement zum Nachweis mindestens eines Analyten in der Probe aufweisen.

Allgemein kann das Nadelelement mindestens eine kapillaraktive Oberflächenstruktur aufweisen, welche im Folgenden auch einfach als Kapillarstruktur bezeichnet wird und welche nicht notwendigerweise vollständig an einer Oberfläche des Nadelelements angeordnet sein muss. Insbesondere kann die Kapillarstruktur mindestens einen Kapillarkanal und/oder mindestens eine Kapillarrinne aufweisen. Aufgrund der Kapillarwirkung der Oberflächenstruktur kann die aus der Einstichstelle austretende Körperflüssigkeit von der Kapillarstruktur aufgenommen werden und beispielsweise zu einem an dem Nadelelement angeordneten Testelement transportiert werden. Die Kapillarstruktur kann ein Aufnahmevolumen bereitstellen, um beispielsweise eine für einen oder mehrere Tests benötigte Menge an Körperflüssigkeit aufzunehmen. Eine solche Oberflächenstruktur ist durch zumindest eine parallel und/oder zumindest teilweise quer zur Längsachse des Nadelelementes verlaufende Kapillarrinne ausbildbar. Im Falle von parallel zur Längsachse des Nadelelementes verlaufenden Kapillarrinnen ist eine Anzahl von 2 bis 6, insbesondere 2 oder 3, solcher Kapillarrinnen bevorzugt. Es ist aber auch möglich, dass beispielsweise bis zu 10 Kapillarrinnen an dem Nadelelement ausgebildet sind. Im Falle beispielsweise einer Hohlkanüle wird die Kapillarstruktur durch die Hohlform der Kanüle ausgebildet.

Vorteilhaft an der Ausbildung solcher Kapillarstrukturen ist insbesondere die Möglichkeit einer gezielten Aufnahme und/oder eines gezielten Transports von Körperflüssigkeit aus oder von der Einstichstelle. Beispielsweise kann ein Transport zu mindestens einem Testelement erfolgen, beispielsweise zu mindestens einem an dem Nadelelement oder zu dem Nadelelement angeordneten oder anordenbaren Testelement. Das Testelement kann fest zu dem Nadelelement und/oder der Kapillarstruktur angeordnet sein, kann jedoch auch beweglich zu diesen gelagert sein, beispielsweise indem nach der Aufnahme der Körperflüssigkeit in die Kapillarstruktur das Testelement auf die Kapillarstruktur zubewegt wird. Vorteilhaft auf die Kapillarwirkung wirkt sich zudem die hydrophile Beschichtung und/oder eine Plasmabehandlung und/oder eine Ausbildung von Nano- und Microstrukturen zumindest in den Kapillarrinnen, aber auch auf der gesamten Oberfläche aus.

Die hydrophile Beschichtung des Nadelelements kann vollständig oder auch teilweise erfolgen. Bevorzugt wird dabei die gesamte Oberfläche des Nadelelementes hydrophil beschichtet und besonders bevorzugt die mindestens eine kapillaraktive Oberflächenstruktur von der Spitze des Nadelelementes bis zur Kontaktstelle Nadelelement/Testelement.

Wird mit dem Nadelelement beispielsweise eine Körperflüssigkeit aufgenommen, so wird die Körperflüssigkeit bevorzugt entlang des Nadelelementes innerhalb von 30-1000 ms pro Millimeter Transportstrecke von der Einstichstelle zum Testelement transportiert, besonders bevorzugt innerhalb von 40-700 ms/mm Transportstrecke und ganz besonders bevorzugt innerhalb von 40-400 ms/mm Transportstrecke. Der Begriff Transportstrecke wird hierbei als diejenige Strecke verstanden, die von der aufgenommenen Probe in dem Nadelelement zurückgelegt wird, vorzugsweise maximal zurückgelegt werden kann. Die gesamte Transpoststrecke kann beispielsweise eine Länge von 0,5 mm bis 10 mm aufweisen, insbesondere 1 mm bis 5 mm. Beispielsweise kann die Transportstrecke eine Gesamtlänge einer Kapillaren, beispielsweise eines Kapillarspalts, in dem Nadelelement sein. Alternativ oder zusätzlich kann sich der Begriff der Transportstrecke auch auf die Länge des Nadelelements von einer Spitze bis hin zu einer Auslassöffnung beziehen, beispielsweise bei einer Kanüle. Es kann mindestens ein Testelement mit mindestens einer Testchemie zum Nachweis des Analyten vorgesehen sein, welches ganz oder teilweise Bestandteil des Nadelelements sein kann, welches jedoch auch ganz oder teilweise separat von dem Nadelelement ausgebildet sein kann. Ist das Testelement nicht Bestandteil des Nadelelements, so kann ein Übertrag der Körperflüssigkeit von dem Nadelelement auch zeitversetzt zu einer Aufnahme der Körperflüssigkeit durch das Nadelelement erfolgen. Bis zu diesem Übertrag kann beispielsweise eine weitere Zeitdauer vergehen, beispielsweise eine Zeitdauer von 0,5 s bis 5 s nach einem Beginn des Einstichs und/oder nach Zurückziehen des Nadelelements aus dem Körpergewebe, insbesondere eine Zeitdauer von 1 s bis 2 s.

Bevorzugt ist das analytische Hilfsmittel ein Nadelelement einer Probennahmevorrichtung zur Entnahme einer Körperflüssigkeit.

Demnach betrifft die vorliegende Erfindung auch ein analytisches Hilfsmittel, wie oben beschrieben, wobei das analytische Hilfsmittel ein Nadelelement einer Probennahmevorrichtung zur Entnahme einer Körperflüssigkeit ist. Weiterhin betrifft die vorliegende Erfindung ein Verfahren zur Herstellung eines analytischen Hilfsmittels, wie oben beschrieben, und ein analytisches Hilfsmittel, herstellbar oder hergestellt durch dieses Verfahren, wobei das analytische Hilfsmittel ein Nadelelement einer Probennahmevorrichtung zur Entnahme einer Körperflüssigkeit ist.

Weiterhin betrifft die vorliegende Erfindung eine Probennahmevorrichtung zur Entnahme einer Körperflüssigkeit. Unter einer Probennahmevorrichtung ist allgemein eine Vorrichtung zu verstehen, welche eingerichtet ist, um eine Probe zum Zweck einer Analyse aufzunehmen. Vorzugsweise ist die Probennahmevorrichtung darüber hinaus auch eingerichtet, um die Probe zu generieren, beispielsweise indem ein Einstich und/oder Einschnitt in eine Hautoberfläche erfolgt, beispielsweise unter Verwendung mindestens eines Nadelelements. Alternativ oder zusätzlich kann die Probennahmevorrichtung auch eingerichtet sein, um mindestens einen Analyten in der Probe nachzuweisen, beispielsweise mittels mindestens eines Testelements, beispielsweise gemäß einer oder mehreren der oben beschriebenen Ausgestaltungen.

Die Probennahmevorrichtung umfasst mindestens ein analytisches Hilfsmittel mit mindestens einer zumindest teilweise mit einer hydrophilen Beschichtung beschichteten Oberfläche, gemäß einer oder mehreren der der oben beschriebenen Ausgestaltungen, mit der Maßgabe, dass das analytische Hilfsmittel ausgewählt ist aus der Gruppe bestehend aus einem Nadelelement und einem Testelement. Bezüglich der möglichen Ausgestaltungen des Nadelelements und/oder des Testelements kann auf die obige Beschreibung und auf die nachfolgend beschriebenen Ausgestaltungen verwiesen werden. Das Nadelelement kann insbesondere mindestens einen Microsampler umfassen, welcher ganz oder teilweise mit der mindestens einen Beschichtung versehen ist, insbesondere im Bereich einer Kapillarstruktur. Das Testelement kann insbesondere mindestens ein mit der hydrophilen Beschichtung versehenes Verteilerelement umfassen, vorzugsweise mindestens ein mit der hydrophilen Beschichtung versehenes Spreitnetz, welches beispielsweise auf mindestens einem Testfeld des Testelements aufliegen kann.

Die Probennahmevorrichtung kann also, wie oben beschrieben, mindestens ein Nadelelement zur Entnahme einer Körperflüssigkeit und/oder mindestens ein Testelement aufweisen, mit dem zumindest ein Analyt in der jeweiligen Körperflüssigkeit qualitativ und/oder quantitativ erfasst werden kann. Das Testelement kann insbesondere mit dem Nadelelement verbunden sein, so dass Körperflüssigkeit von dem Nadelelement zu dem Testelement, beispielsweise einer Probenaufgabestelle oder einer Probenaufgabenoberfläche des Testelements, gelangen kann. Alternativ oder zusätzlich kann das Testelement jedoch auch als separates Testelement ausgestaltet sein oder auf andere Weise in oder an der Probennahmevorrichtung aufgenommen sein, beispielsweise in mindestens einer Kammer, in der beispielsweise auch das Nadelelement gelagert sein kann. Es kann ein Transfermechanismus vorgesehen sein, der die von dem Nadelelement aufgenommene Körperflüssigkeit zu dem Testelement transferiert. Dies kann beispielsweise dadurch erfolgen, dass mittels eines Aktors oder eines anderen Mechanismus das Nadelelement mit der Körperflüssigkeit derart an das Testelement angenähert wird, dass die Körperflüssigkeit zumindest teilweise von dem Nadelelement auf das Testelement, beispielsweise ein Testfeld des Testelements, übertragen wird. Beispielsweise kann zu diesem Zweck das Nadelelement und/oder mindestens eine Kapillarstruktur des Nadelelements auf das Testfeld aufgelegt werden. Auch andere Ausgestaltungen sind jedoch möglich. Damit die Körperflüssigkeit aus der kapillaraktiven Oberflächenstruktur in das Testelement übertreten kann, ist das Testelement vorzugsweise an einer Anbindungsstelle zwischen Testelement und Oberflächenstruktur so auszubilden, dass die Körperflüssigkeit aus der Oberflächenstruktur in das Testelement eingesaugt wird. Dazu ist das Testelement zweckmäßig mit einer kapillaraktiven Komponente, wie z.B. einem Saugvlies oder einem Faserbündel, auszustatten, dessen kapillare Wirksamkeit größer ist als die Kapillarität der Oberflächenstruktur. Alternativ oder zusätzlich kann jedoch eine Ausgestaltung auch einfach derart erfolgen, dass die Kapillare mit der Chemie kontaktiert wird, beispielsweise indem die Kapillare auf die Chemie gelegt wird, wobei optional auch ein Druck ausgeübt werden kann. Des Weiteren sollte ein Kontakt zwischen der Oberflächenstruktur der Kanüle und der kapillaraktiven Komponente des Testelements sichergestellt sein.

Des Weiteren kann die Probennahmevorrichtung mindestens einen Sensor aufweisen. Dieser Sensor kann beispielsweise elektrochemisch und/oder optisch ausgebildet sein. Bei einem elektrochemischen Sensor kann aufgrund einer katalytischen Wirkung der Testchemie eine zu bestimmende Substanz der Körperflüssigkeit umgesetzt und diese Umsetzung elektrochemisch vermessen werden, während bei einem optischen Sensor ein Reaktionsprodukt der Testchemie mit einer in der Körperflüssigkeit enthaltenen Substanz mit Licht einer definierten Wellenlänge bestrahlt wird und beispielsweise ein Grad einer Absorption dieses Lichtes und/oder ein Grad einer Reflektion des Lichts und/oder die Fluoreszenz der Probe vermessen wird.

Weiterhin kann die Probennahmevorrichtung mit einem Leitungselement ausgestattet sein, insbesondere einem elektrischen Leitungselement. Im Falle eines elektrochemischen Sensors kann dieses Leitungselement eine auf einer Kunststofffolie aufgetragene Metallschicht sein, und/oder eine Metallfolie oder ein Metalldraht, der gegebenenfalls durch z.B. ein Spritzgussverfahren in die jeweilige Komponente eingespritzt ist. Auch die Ausbildung des Leitungselements aus leitfähigem Kunststoff oder auf andere Weise, beispielsweise mittels leitfähiger Tinte, ist realisierbar. Die Probennahmevorrichtung kann ebenfalls mindestens eine elektrische und/oder optische Kontaktstelle aufweisen, über die die Probennahmevorrichtung mit einem peripheren Gerät gekoppelt werden kann. Somit ist die Probennahmevorrichtung über eine elektrische Kontaktstelle mit Strom versorgbar und es können über eine solche elektrische Kontaktstelle Informationen übertragen werden. Über eine optische Kontaktstelle lässt sich Licht zwischen einem peripheren Gerät und der Probennahmevorrichtung übertragen. Im Falle eines optischen Sensors kann eine solche Probennahmevorrichtung sowohl mit einer elektrischen als auch mit einer optischen Kontaktstelle ausgestattet sein.

Bevorzugt kann die Probennahmevorrichtung ganz oder teilweise als Einweg- und/oder Mehrwegartikel ausgebildet sein, wobei im Falle eines Einwegartikels die Probennahmevorrichtung nach einmaligem Benutzen entsorgt wird, während im Falle eines Mehrwegartikels eine mehrfache Benutzung bestimmungsgemäß vorgesehen ist. Es ist ebenfalls eine Magazinierung einer oder mehrerer Probennahmevorrichtungen und/oder von Teilen derselben analog z.B. zu der Magazinierung von Lanzetten bei Stechhilfen denkbar, beispielsweise indem mehrere Probennahmevorrichtungen als ganze magaziniert werden und/oder indem eine Probennahmevorrichtung beispielsweise mehrere Testelemente und/oder mehrere Nadelelemente in magazinierter Form enthält.

Wie oben beschrieben, kann das analytische Hilfsmittel, bevorzugt das Nadelelement und/oder das Testelement, zumindest teilweise aus einem Metall und/oder einer Metalllegierung und/oder einem Metalloxid und/oder einem Mischmetalloxid und/oder einem Metallmischoxid hergestellt sein. Dabei sollte das Material des Nadelelementes und/oder des Testelements inert sein, insbesondere gegenüber der zu entnehmenden Körperflüssigkeit, sowie biokompatibel, als auch mechanisch beanspruchbar und auch leicht zu sterilisieren. Bevorzugt kann das Nadelelement aus Chirurgenstahl hergestellt sein, wobei dieser Chirurgenstahl mit einer Beschichtung aus Metalloxiden versehen sein kann. Alternativ oder zusätzlich zu einem metallischen Werkstoff kann das Nadelelement jedoch auch ganz oder teilweise aus einem anderen Werkstoff hergestellt sein oder einen anderen Werkstoff umfassen, beispielsweise einen Kunststoffwerkstoff und/oder einen keramischen Werkstoff. Insbesondere sind auch Ausführungsformen im Rahmen der Erfindung umfasst, bei denen das Nadelelement an sich, wie oben beschrieben, mit einem Metall und/oder einer Metalllegierung und/oder einem Metalloxid und/oder einem Mischmetalloxid und/oder einem Metallmischoxid beschichtet ist, wobei auf dieser Oberfläche, beispielsweise einer Oberfläche dieser Beschichtung, die hydrophile Beschichtung aufgebracht wird.

Das erfindungsgemäße analytische Hilfsmittel und die erfindungsgemäßen Verfahren weisen gegenüber bekannten analytischen Hilfsmittel und Verfahren eine Vielzahl von Vorteilen auf. So wurde überraschenderweise herausgefunden, dass die hydrophile Beschichtung mit Nanopartikeln der oben beschriebenen Art einerseits zu Oberflächeneigenschaften mit hervorragender Hydrophilie führt. Andererseits zeichnet sich überraschenderweise die Beschichtung durch eine gute Langzeitstabilität aus, so dass beispielsweise eine stabile Lagerung über einen längeren Zeitraum hinweg gewährleistet werden kann. Insbesondere lässt sich zeigen, dass auch ein direkter oder indirekter Kontakt mit verschiedenen Werkstoffen des analytischen Hilfsmittels, wie beispielsweise Kunststoffen, Klebstoffen oder Metallen, die hydrophilen Eigenschaften der Beschichtung nicht oder nur unwesentlich beeinträchtigen. Auch eine Belastung durch eine Sterilisation, beispielsweise eine Strahlenbelastung durch β-Strahlen und/oder γ-Strahlen und/oder eine Belastung durch chemische Desinfektionsmittel wie beispielsweise Ethylenoxid, beeinträchtigen die hydrophilen Eigenschaften der Beschichtung nicht oder nur unwesentlich. Somit lässt sich, bei Aufrechterhaltung der hydrophilen Oberflächeneigenschaften, eine Langzeitstabilität von 6-12 Monaten oder mehr problemlos realisieren.

Die erfindungsgemäß verwendete Beschichtung ist zudem äußerst resistent gegenüber Verunreinigungen und kontaminiert selbst beispielsweise eine aufgenommene Probe nicht. Das analytische Hilfsmittel kann dementsprechend optional auch mehrfach verwendet werden. Insbesondere kann eine erfindungsgemäß eingesetzte Beschichtung in Kapillarelementen und/oder Kanälen des analytischen Hilfsmittels eingesetzt werden, wobei diese Elemente durch die erfindungsgemäße Beschichtung eine langlebige Hydrophilie aufweisen.

Zudem lässt sich die erfindungsgemäß verwendete Beschichtung auf einfache und zuverlässige Weise herstellen, beispielsweise unter Verwendung des erfindungsgemäßen Verfahrens gemäß einer oder mehreren der oben beschriebenen Ausgestaltungen oder der nachfolgend noch näher beschriebenen Ausführungsformen. Insbesondere lässt sich eine Applikation der Oberflächenbeschichtung beispielsweise aus wässriger Lösung und/oder aus wässriger Dispersion durchführen. Eine derartige Applikation ist verfahrenstechnisch einfach realisierbar.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt.

Es zeigt:

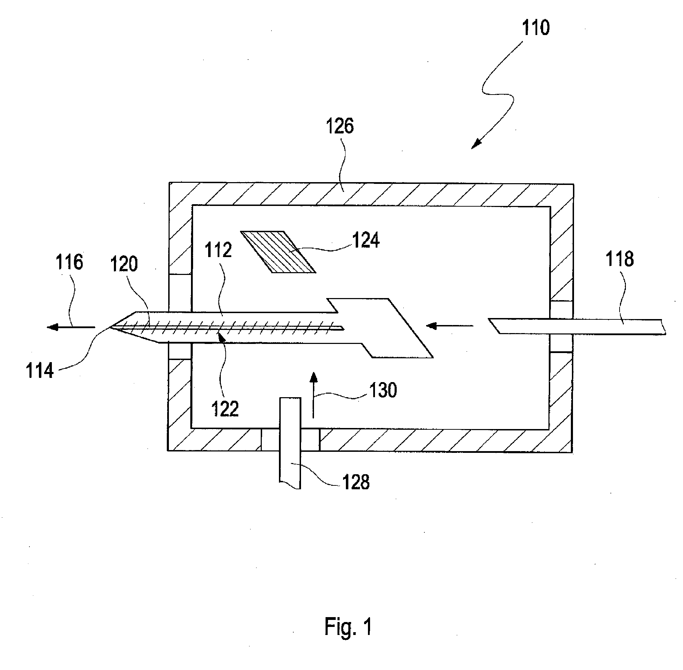

- Figur 1: ein Ausführungsbeispiel einer Probennahmevorrichtung.

### Ausführungsbeispiele

In Figur 1 ist in einer stark schematisierten Darstellung ein Ausführungsbeispiel einer möglichen erfindungsgemäßen Probennahmevorrichtung 110 in Schnittdarstellung von der Seite dargestellt. Die Erfindung kann jedoch auch auf eine Vielzahl anderer Arten von Probennahmevorrichtungen und analytischen Hilfsmitteln angewandt werden.

Die Probennahmevorrichtung 110 umfasst ein in Figur 1 schematisch dargestelltes Nadelelement 112 mit einer Spitze 114, welche bei einer Bewegung des Nadelelements 112 in einer Einstichrichtung 116 eine Hautpartie eines Benutzers perforiert. Die Bewegung des Nadelelements 112 kann beispielsweise durch einen optionalen Aktor 118 der Probennahmevorrichtung 110 angetrieben werden, welcher in Figur 1 schematisch dargestellt ist und welcher auf verschiedene, dem Fachmann bekannte Weisen ausgestaltet werden kann. Der Aktor 118 kann einen Vorschub des Nadelelements 112 in Einstichrichtung 116 bewirken und optional auch eine Rückbewegung des Nadelelements 112 entgegen der Einstichrichtung.

Das Nadelelement 112 kann in dem dargestellten Ausführungsbeispiel oder auch in anderen Ausführungsbeispielen weiterhin optional mindestens eine kapillaraktive Oberflächenstruktur in Form beispielsweise einer Kapillarstruktur 120 umfassen, beispielsweise mindestens einen Kapillarspalt, welcher sich insbesondere parallel zur Einstichrichtung 116 erstrecken kann. Auch andere Ausgestaltungen der Kapillarstruktur 120 sind möglich. Die Kapillarstruktur 120 kann beispielsweise zur Aufnahme und/oder zum Transport einer Probe der Körperflüssigkeit bei einem Probennahmevorgang dienen, wenn das Nadelelement 112 in ein Körpergewebe eindringt. Beispielsweise kann ein Transport oder eine Aufnahme der Probe über Kapillarkräfte erfolgen.

Das Nadelelement 112 umfasst weiterhin mindestens eine hydrophile Beschichtung 122, vorzugsweise im Bereich der Kapillarstruktur 120. Mögliche Ausgestaltungen dieser hydrophilen Beschichtung 122 sowie mögliche Verfahren und Beispiele zu deren Herstellung werden unten noch näher erläutert.

Die Probennahmevorrichtung 110 kann weiterhin mindestens ein Testelement 124 umfassen, beispielsweise mit mindestens einer Testchemie, zum Nachweis mindestens eines Analyten in der Körperflüssigkeit. Beispielsweise umfasst dieses Testelement mindestens ein Testfeld, also mindestens eine mit der Testchemie beschichtete Fläche. In Figur 1 ist das Testelement 124 symbolisch als von dem Nadelelement 112 getrennt ausgebildetes Testelement 124 dargestellt, welches beispielsweise in oder an einem Gehäuse 126 der Probennahmevorrichtung 110, beispielsweise einem Magazingehäuse, angeordnet sein kann. Beispielsweise kann nach einer Probenaufnahme das Nadelelement 112 mit der Kapillarstruktur 120 an das Testelement 124 angenähert werden, um eine Probe der Körperflüssigkeit oder einen Teil derselben aus der Kapillarstruktur 120 auf das Testelement 124 zu übertragen. Zu diesem Zweck kann beispielsweise in der Probennahmevorrichtung 110 ein Aktor 128 vorgesehen sein, welcher das Nadelelement 112 oder einen Teil derselben nach der Probenaufnahme in einer Bewegungsrichtung 130 auf das Testelement 124 zu bewegt. Alternativ oder zusätzlich sind jedoch auch andere Möglichkeiten gegeben, um die Körperflüssigkeit aus der Kapillarstruktur 120 und/oder von dem Nadelelement 112 auf das Testelement 124 zu übertragen, beispielsweise indem das Nadelelement 112 während einer Rückbewegung nach der Probenahme durch eine geeignete Bahn an dem Testelement 124 vorbeigeführt wird. Wiederum alternativ oder zusätzlich ist es jedoch auch möglich, das optionale Testelement ganz oder teilweise in das Nadelelement 112 zu integrieren, beispielsweise an einem Ende der Kapillarstruktur 120.

Im Folgenden werden verschiedene Ausführungsbeispiele betreffend die Herstellung hydrophil beschichteter analytischer Hilfsmittel erläutert.

### Beispiele:

### Beispiel 1: Beschichtung der Kapillaren und Überprüfung der Haltbarkeiten von Hydrophilierungsmitteln

### Verwendete Materialien:

- Kapillaren mit Querschnitten von 120x80 µm
- Granulat Makrolon^{®} 2458 (Bayer) Materialien für Beschichtung:
   1. Ar-Plasma
   2. Heparin
   3. Carbopol^{®} 971PNF (Na-Salz einer teilvernetzten hochmolekularen Polyacrylsäure) (kommerziell erhältlich beispielsweise von Lubrizol)
   4. Mega 8 (Octanoyl-N-methylglucamid) (kommerziell erhältlich beispielsweise von Dojindo)
   5. DONS (Dioctylnatriumsuccinat) (kommerziell erhältlich beispielsweise von Cytec Industries B.V.)
   6. PVA 28-99 (Polyvinylalkohol) (kommerziell erhältlich beispielsweise von Fluka)
   7. PVP K15 (Polyvinylpyrrolidon) (kommerziell erhältlich beispielsweise von Aldrich)
   8. Bindzil^{®} CC30 (nano-Silica-Dispersion) (Akzo Nobel)
   9. Ludox^{®} AM-30 (nano-Silica-Dispersion) (kommerziell erhältlich beispielsweise von Aldrich)
   10. Ludox^{®} AS-30 (nano-Silica-Dispersion) (kommerziell erhältlich beispielsweise von Aldrich)
   11. Bindzil^{®} 2034DI (nano-Silica-Dispersion) (kommerziell erhältlich beispielsweise Akzo-Nobel bzw. eka)
   12. CMC (Carboxymethylcellulose, MW = 700 000 g/mol, Substitutionsgrad DS 0.9) (kommerziell erhältlich beispielsweise von Aldrich)
   13. Bindzil^{®} CC301 (nano-Silica-Dispersion) (Akzo Nobel)
   14. Bindzil^{®} CC401 (nano-Silica-Dispersion) (Akzo Nobel)
   15. Bindzil^{®} 30/360 (nano-Silica-Dispersion) (Akzo Nobel)

### Allgemeine Vorschrift 1(a) zur Beschichtung der Kapillaren

Die Beschichtung erfolgt, indem man die wässrigen Dispersionen oder Lösungen der Beschichtungsmaterialien allein durch die Kapillarkräfte in die Kapillaren mit Querschnitten von 120x80 µm hineinfließen lässt, bis die Kapillaren vollständig gefüllt sind (ca. 35 nanoliter). Anschließend wird die Beschichtung mit einem Föhn bei Raumtemperatur oder durch Erwärmen bis 140°C kurz getrocknet.

Vor der Beschichtung werden die Kapillaren durch eine Plasmabehandlung benetzbar gemacht.

### Allgemeine Vorschrift 1(b) zur Plasmabehandlung der Kapillaren

Die Kapillaren werden für 30 s in einem Plasmaofen (Hersteller Plateg) bei 50 mbar Argondruck und 400 Watt Mikrowellenleistung behandelt.

### Allgemeine Vorschrift 1(c) zur Sterilisierung

Die Sterilisierung erfolgt durch Behandlung mit Betastrahlung, 25 kGRay.

### Beispiel 1.1 Haltbarkeiten von Hydrophilierungsmitteln in Gegenwart von Makrolon

Die Haltbarkeiten der verschiedenen Hydrophilierungsmittel in Gegenwart von flüchtigen Bestandteilen von Verpackungsmaterialien wurden am Beispiel von Makrolon^{®} als solches Material überprüft, indem je 6 gemäß allgemeiner Vorschrift 1(a) beschichtete Kapillaren, die vor der Beschichtung gemäß allgemeiner Vorschrift 1(b) Plasma-behandelt wurden, mit je 4 g Granulat Makrolon 2458 oder ohne Granulat in einem PET-Beutel eingeschweißt und für die angegebene Zeit gelagert wurden. Wenn nicht anders angegeben, wurden alle Beutel mit 25kGray Beta-Strahlung sterilisiert. Gemessen wurde die Zeit für eine Füllung über eine Länge von 4 mm. Die Messung erfolgte mit Heparin-Blut, HK 44. Die Ergebnisse sind in Tabelle 1 dargestellt.

**Tabelle 1**

| Material | Konzentration der verwendeten Lösung/Dispersion in Wasser] (w/w) | direkt nach Sterilisierung | 8 Tage / 75°C sterilisiert ohne Makrolon | 8 Tage / 75°C sterilisiert mit Makrolon | 21 Tage / 75°C sterilisiert mit Makrolon |
|---|---|---|---|---|---|
| Ar-Plasma* | --- | < 300 ms (ohne Sterisilierung) | füllt nicht | füllt nicht | --- |
| Heparin* | 0,05% | --- | 300 ms | füllt nicht | --- |
| Carbopol^{®} 971PNF* | 0,1% | --- | 450ms | 625 ms | ca. 2100 ms |
| Mega 8* | 0,05% | füllt nicht | füllt nicht | füllt nicht | --- |
| DONS* | 0,05% | füllt nicht | füllt nicht | füllt nicht | --- |
| PVA 28-99* | 0,05% | ca. 2000 ms | > 2500 ms | ca. 2000 ms | --- |
| PVP K15* | 0,05% | --- | 545 ms | 680 ms | ----- |
| Bindzil^{®} CC30 | 0,5% | --- | 225 ms | 200 ms | 210 ms |
| Ludox^{®} AM-30 | 0,5% | --- | 342 ms | 400 ms | 320 ms |
| Ludox^{®} AS-30 | 0,5% | --- | 270 ms | 240 ms | 260 ms |
| Bindzil^{®} 2034DI | 0,5% | --- | 305 ms | 240 ms | 300 ms |

| | | | | | |
|---|---|---|---|---|---|
| * nicht erfindungsgemäß | | | | | |

Die Ergebnisse belegen, dass die erfindungsgemäßen Beschichtungen, auch nach Lagerung in PET Beuteln in Anwesenheit von Makrolon, eine Füllzeit von weniger als 400 ms für 4 mm Länge bei einem Querschnitt von 120x80 µm ermöglichen.

### Beispiel 1.2 Haltbarkeiten von Hydrophilierungsmitteln in Gegenwart von Zylar

Die Haltbarkeiten der verschiedenen Hydrophilierungsmittel in Gegenwart von flüchtigen Bestandteilen von Verpackungsmaterialien wurden weiterhin am Beispiel von Zylar^{®}, welches im Vergleich zu Makrolon einen erhöhten Anteil an flüchtigen Substanzen enthält, überprüft, indem je 6 gemäß allgemeiner Vorschrift 1(a) beschichtete Kapillaren, die vor der Beschichtung gemäß allgemeiner Vorschrift 1(b) Plasma-behandelt wurden, zusammen mit Spritzgussteilen aus dem Material Zylar 220 (MBS-Copolymer, Hersteller: Ineos-Nova, ca. 1,5 mm dick, 4 g schwer) gelagert wurden. Gemessen wurde die Zeit für eine Füllung über eine Länge von 4 mm. Die Messung erfolgte mit Heparin-Blut, HK 44. Die Ergebnisse sind in Tabelle 2 dargestellt.

**Tabelle 2**

| Material | Konzentration Silica in Wasser w/w | Direkt nach Sterilisation, mit Zylar 220 | 1 Woche 75°C mit Zylar 220 | 2 Wochen 75°C mit Zylar 220 |
|---|---|---|---|---|
| Bindzil^{®} CC301 | 0,5% | <300 | <500 | <700 |
| Bindzil^{®} CC30 | 0,5% | <300 | <500 | <700 |
| Bindzil^{®} CC401 | 0,5% | <300 | <500 | <700 |
| Ludox^{®} AS-30 | 0,5% | <300 | Keine Füllung | Keine Füllung |
| Ludox^{®} AM-30 | 0,5 % | <300 | Keine Füllung | Keine Füllung |
| Bindzil^{®} 30/360 | 0,5% | <300 | Keine Füllung | Keine Füllung |

Bei Anwesenheit von Zylar zeigen die Beschichtungen mit mit organischen Gruppen modifizierten Silica-Nanopartikeln Vorteile gegenüber den Silica-Nanopartikeln ohne organische Modifikationen.

### Beispiel 1.3: Vergleichsbeispiel: Haltbarkeiten von Hydrophilierungsmitteln in Gegenwart von Zylar bei nicht-erfindungsgemäßer Beschichtung:

Als Vergleichsbeispiel zum Vergleich erfindungsgemäßer Beschichtungen mit nicht-erfindungsgemäßen Beschichtungen wurde das unter Beispiel 1.2 beschriebene Ausführungsbeispiel nochmals mit nicht-erfindungsgemäßen Beschichtungen wiederholt. Dabei wurden als Beschichtungsmaterial Mischungen aus Carboxymethylcellulose mit DONS aufgebracht, also Beschichtungen, die in der EP 2 014 727 A1 beschrieben werden. Die Beschichtung erfolgte, indem zunächst eine wässrige Lösung der Carboxymethylcellulose hergestellt wurde und diese mit der entsprechenden Menge DONS versetzt wurde. Die erhaltene wässrige Lösung wurde zur Beschichtung gemäß allgemeiner Vorschrift 1(a) verwendet.

Die Haltbarkeiten der jeweiligen Mischungen von CMC und DONS in Gegenwart von flüchtigen Bestandteilen von Verpackungsmaterialien wurde wiederum, entsprechend Beispiel 1.2, am Beispiel von Zylar ermittelt.

Die Ergebnisse sind in Tabelle 3 dargestellt.

**Tabelle 3**

| Material (Massenverhältnis) | Konzentration in Wasser w/w | Direkt nach Sterilisation, mit Zylar 220 | 1 Woche 75°C mit Zylar 220 |
|---|---|---|---|
| CMC/DONS (20:1) | 0,05 % | <300 | Keine Füllung |
| CMC/DONS (10:1) | 0,05 % | <300 | Keine Füllung |

Bei Anwesenheit von Zylar füllten die Kapillaren mit den Beschichtungen mit CMC und DONS bereits nach 1 Woche nicht mehr.

### Bezugszeichen

- 110: Probennahmevorrichtung
- 112: Nadelelement
- 114: Spitze
- 116: Einstichrichtung
- 118: Aktor
- 120: Kapillarstruktur
- 122: hydrophile Beschichtung
- 124: Testelement
- 126: Gehäuse
- 128: Aktor
- 130: Bewegungsrichtung

## Patentansprüche

1. Analytisches Hilfsmittel, umfassend eine zumindest teilweise mit einer hydrophilen Beschichtung beschichtete Oberfläche,
**dadurch gekennzeichnet, dass**
die hydrophile Beschichtung Nanopartikel mit Siliciumdioxidstruktur und einer mittleren Partikelgröße bestimmt gemäß DIN ISO 22412:2008, im Bereich von 1 bis 500 nm enthält und die Nanopartikel Gruppen der Struktur (I) oder (II) umfassen 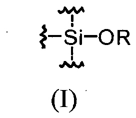 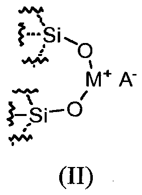 wobei in jeder der Gruppen der Struktur (I), unabhängig voneinander, R ausgewählt ist aus der Gruppe bestehend aus H, einem Metall enthaltendem Ion, 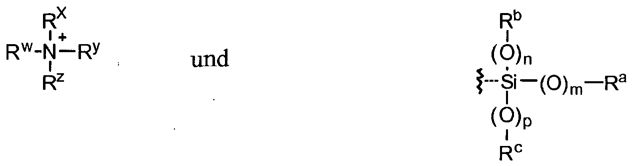 und wobei R^{w}, R^{x}, R^{y} und R^{z}, unabhängig voneinander, ausgewählt sind aus H und Alkyl,
und wobei R^{a}, R^{b} und R^{c}, unabhängig voneinander, optional substituierte Reste, ausgewählt aus der Gruppe bestehend aus H, Alkyl, Aryl, Heteroaryl, Cycloalkyl, Cycloheteroalkyl, Alkenyl und Alkoxyalkyl, sind,
und wobei n, m und p, unabhängig voneinander, 0 oder 1 sind,
und wobei M⁺ ein Metallion und A- ein physiologisch verträgliches Anion ist.

2. Analytisches Hilfsmittel nach Anspruch 1, wobei R ausgewählt ist aus der Gruppe bestehend aus H, einem Erdalkalimetallion, einem Alkalimetallion, 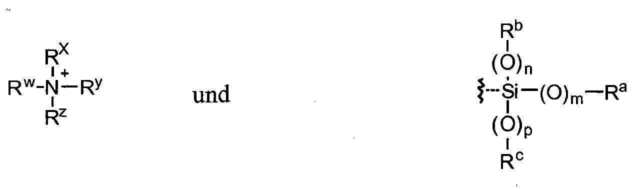 und wobei M⁺ = Al⁺ ist.

3. Analytisches Hilfsmittel nach Anspruch 1 oder 2, wobei die Siliciumdioxidstruktur der Nanopartikel mindestens ein Fremdatom, ausgewählt aus der Gruppe bestehend aus Sn, Ti, Zr, Ge, In, Ga, Al und B, aufweist, bevorzugt wobei das Fremdatom Al ist.

4. Analytisches Hilfsmittel nach einem der Ansprüche 1 bis 3, wobei die Nanopartikel eine mittlere Partikelgröße im Bereich von 1 bis 100 nm, bevorzugt im Bereich von 1 bis 50 nm, weiter bevorzugt im Bereich von 3 nm bis 20 nm, aufweisen.

5. Analytisches Hilfsmittel nach einem der Ansprüche 1 bis 4, wobei 10 bis 40 % aller Gruppen R 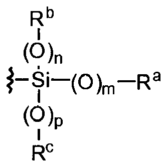 sind.

6. Analytisches Hilfsmittel nach Anspruch 4, wobei die Gruppe der Formel (I) eine Struktur der Formel (Ia) oder (Ib) aufweist: 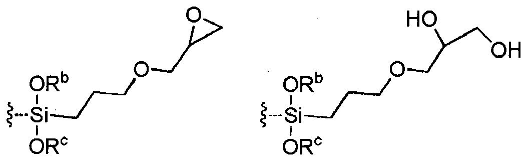 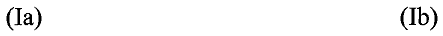 wobei R^{b} und R^{c}, unabhängig voneinander, ausgewählt sind aus der Gruppe bestehend aus H und Alkyl.

7. Analytisches Hilfsmittel nach einem der Ansprüche 1 bis 5, wobei die hydrophile Beschichtung aus den Nanopartikeln mit Siliciumdioxidstruktur besteht.

8. Analytisches Hilfsmittel nach einem der Ansprüche 1 bis 7, wobei die zumindest teilweise mit der hydrophilen Beschichtung beschichtete Oberfläche des analytischen Hilfsmittels zumindest teilweise aus einem Metall und/oder einer Metalllegierung und/oder einem Metalloxid und/oder einem Mischmetalloxid besteht.

9. Analytisches Hilfsmittel nach einem der Ansprüche 1 bis 7, wobei die Nanopartikel eine mittlere Partikelgröße im Bereich von 1 bis 100 nm aufweisen und wobei die hydrophile Beschichtung eine Dicke, insbesondere eine mittlere Dicke, im Bereich von höchstens 500 nm, vorzugsweise von höchstens 300 nm und insbesondere von höchstens 100 nm aufweist.

10. Analytisches Hilfsmittel nach einem der Ansprüche 1 bis 9, wobei die Oberfläche der hydrophilen Beschichtung einen Kontaktwinkel mit Wasser kleiner 50°, gemessen vorzugsweise gemäß DIN 55660.2, aufweist.

11. Analytisches Hilfsmittel nach einem der Ansprüche 1 bis 10, wobei das analytische Hilfsmittel ausgewählt ist aus der Gruppe bestehend aus einem Nadelelement, insbesondere einer Lanzette, einer Kapillare, insbesondere einem Kapillarspalt, einer Kanüle, einem Testelement zum Nachweis mindestens eines Analyten in einer Körperflüssigkeit und einem Verteilerelement zur Verteilung einer aufgenommenen Probe einer Körperflüssigkeit, insbesondere einem, gegebenenfalls mit einem Metall und/oder einer Metalllegierung und/oder einem Metalloxid und/oder einem Mischmetalloxid beschichteten, Spreitnetz oder einer, gegebenenfalls mit einem Metall und/oder einer Metalllegierung und/oder einem Metalloxid und/oder einem Mischmetalloxid beschichteten, Kunststofffolie.

12. Analytisches Hilfsmittel nach einem der Ansprüche 1 bis 11, wobei das analytische Hilfsmittel ein Nadelelement (112) einer Probennahmevorrichtung (110) zur Entnahme einer Körperflüssigkeit ist.

13. Verfahren zur Herstellung eines analytischen Hilfsmittels, umfassend eine Oberfläche, die mindestens teilweise mit einer hydrophilen Beschichtung beschichtet ist, wobei das Verfahren umfasst:
(a) Bereitstellen des analytischen Hilfsmittels,
(b) Beschichten des analytischen Hilfsmittels durch Inkontaktbringen des analytischen Hilfsmittels mit einem Gemisch G umfassend mindestens ein Dispersionsmittel und Nanopartikel mit Siliciumdioxidstruktur, die eine mittlere Partikelgröße bestimmt gemäß DIN ISO 22412:2008, im Bereich von 1 bis 500 nm aufweisen,
wobei die Nanopartikel Gruppen der Struktur (I) oder (II) umfassen 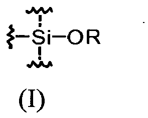 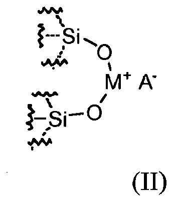 wobei in jeder der Gruppen der Struktur (I), unabhängig voneinander, R ausgewählt ist aus der Gruppe bestehend aus H, einem Metall enthaltendem Ion, 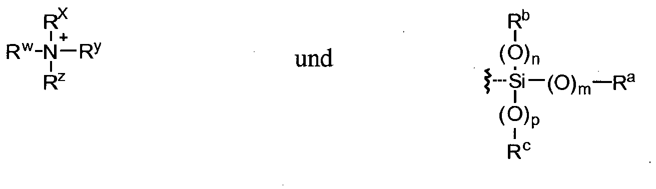 und wobei R^{w}, R^{x}, R^{y} und R^{z}, unabhängig voneinander, ausgewählt sind aus H und Alkyl,
und wobei R^{a}, R^{b} und R^{c}, unabhängig voneinander, optional substituierte Reste, ausgewählt aus der Gruppe bestehend aus H, Alkyl, Aryl, Heteroaryl, Cycloalkyl, Cycloheteroalkyl, Alkenyl und Alkoxyalkyl, sind,
und wobei n, m und p, unabhängig voneinander, 0 oder 1 sind,
und wobei M⁺ ein Metallion und A- ein physiologisch verträgliches Anion ist,
(c) Trocknen des gemäß (b) erhaltenen analytischen Hilfsmittels, und
(d) optional Sterilisieren des gemäß (c) erhaltenen analytischen Hilfsmittels,
wobei das Inkontaktbringen in (b) bevorzugt mittels Tauchbeschichtung und/oder Sprühbeschichtung und/oder Kontaktbeschichtung erfolgt, und wobei das analytische Hilfsmittel und/oder die Oberfläche des analytischen Hilfsmittels bevorzugt zumindest teilweise aus einem Metall und/oder einer Metalllegierung und/oder einem Metalloxid und/oder einem Mischmetalloxid und/oder einem Metallmischoxid besteht.

14. Verfahren nach Anspruch 13, wobei die zu beschichtende Oberfläche des analytischen Hilfsmittels vor Schritt (b) mit mindestens einem Ätzmittel und/oder mit Plasma behandelt wird.

15. Verfahren nach Anspruch 13 oder 14, wobei das mindestens eine Dispersionsmittel Wasser ist und wobei das Gemisch G bevorzugt einen pH-Wert im Bereich von 2 bis 10 aufweist.

16. Verfahren nach einem der Ansprüche 13 bis 15, wobei die Nanopartikel Gruppen der Struktur (I) umfassen, und wobei 10 bis 40 % aller Gruppen R 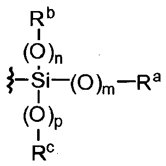 sind.

17. Probennahmevorrichtung (110) zur Entnahme einer Körperflüssigkeit, enthaltend mindestens ein analytisches Hilfsmittel nach einem der vorhergehenden, ein analytisches Hilfsmittel betreffenden Ansprüche, mit der Maßgabe, dass das analytische Hilfsmittel ausgewählt ist aus der Gruppe bestehend aus einem Nadelelement (112) und einem Testelement.

18. Verwendung von Nanopartikeln mit Siliciumdioxidstruktur und einer mittleren Partikelgröße bestimmt gemäß DIN ISO 22412:2008, im Bereich von 1 bis 500 nm als hydrophile Beschichtung (122) für eine Oberfläche eines analytischen Hilfsmittels, weiter bevorzugt als hydrophile Beschichtung (122) für ein Nadelelement (112) einer Probennahmevorrichtung (110) zur Entnahme einer Körperflüssigkeit, wobei die Nanopartikel Gruppen der Struktur (I) oder (II) umfassen 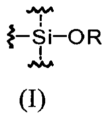 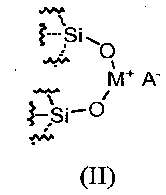 wobei in jeder der Gruppen der Struktur (I), unabhängig voneinander, R ausgewählt ist aus der Gruppe bestehend aus H, einem Metall enthaltendem Ion, 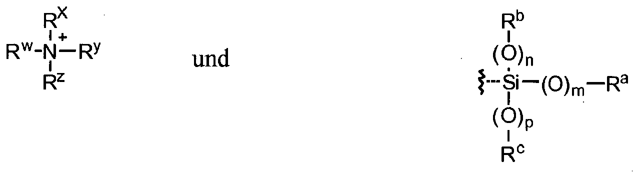 und wobei R^{w}, R^{x}, R^{y} und R^{z}, unabhängig voneinander, ausgewählt sind aus H und Alkyl,
und wobei R^{a}, R^{b} und R^{c}, unabhängig voneinander, optional substituierte Reste, ausgewählt aus der Gruppe bestehend aus H, Alkyl, Aryl, Heteroaryl, Cycloalkyl, Cycloheteroalkyl, Alkenyl und Alkoxyalkyl, sind,
und wobei n, m und p, unabhängig voneinander 0 oder 1 sind,
und wobei M⁺ ein Metallion und A- ein physiologisch verträgliches Anion ist.

## Claims

1. Analytical aid, comprising a surface coated at least partially with a hydrophilic coating, **characterized in that** the hydrophilic coating contains nanoparticles with silica structure and an average particle size, determined according to DIN ISO 22412:2008, in the range from 1 to 500 nm and the nanoparticles comprise groups of structure (I) or (II) 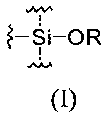 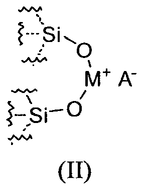 wherein in each of the groups of structure (I), independently of one another, R is selected from the group consisting of H, a metal-containing ion, 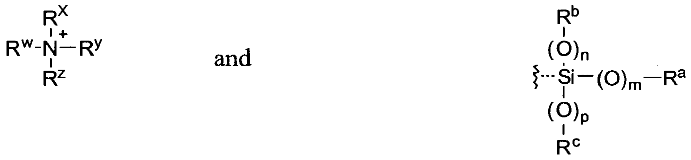 and wherein R^{w}, R^{x}, R^{y} and R^{z}, independently of one another, are selected from H and alkyl,
and wherein R^{a}, R^{b} and R^{c}, independently of one another, are optionally substituted residues, selected from the group consisting of H, alkyl, aryl, heteroaryl, cycloalkyl, cycloheteroalkyl, alkenyl and alkoxyalkyl,
and wherein n, m and p, independently of one another, are 0 or 1,
and wherein M⁺ is a metal ion and A- is a physiologically compatible anion.

2. Analytical aid according to Claim 1, wherein R is selected from the group consisting of H, an alkaline-earth metal ion, an alkali metal ion, 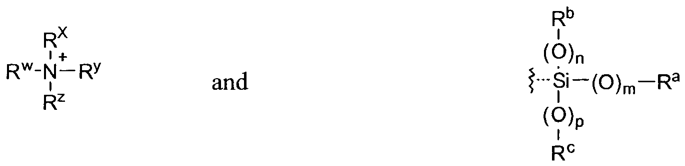 and wherein M⁺ = Al⁺.

3. Analytical aid according to Claim 1 or 2, wherein the silica structure of the nanoparticles has at least one foreign atom, selected from the group consisting of Sn, Ti, Zr, Ge, In, Ga, Al and B, preferably wherein the foreign atom is Al.

4. Analytical aid according to one of Claims 1 to 3, wherein the nanoparticles have an average particle size in the range from 1 to 100 nm, preferably in the range from 1 to 50 nm, more preferably in the range from 3 nm to 20 nm.

5. Analytical aid according to one of Claims 1 to 4, wherein 10 to 40% of all groups R are

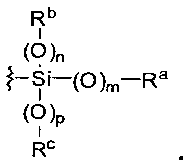

6. Analytical aid according to Claim 4, wherein the group of formula (I) has a structure of formula (Ia) or (Ib): 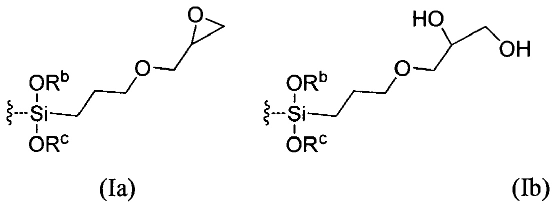 wherein R^{b} and R^{c}, independently of one another, are selected from the group consisting of H and alkyl.

7. Analytical aid according to one of Claims 1 to 5, wherein the hydrophilic coating consists of the nanoparticles with silica structure.

8. Analytical aid according to one of Claims 1 to 7, wherein the surface of the analytical aid, coated at least partially with the hydrophilic coating, consists at least partially of a metal and/or a metal alloy and/or a metal oxide and/or a mixed metal oxide.

9. Analytical aid according to one of Claims 1 to 7, wherein the nanoparticles have an average particle size in the range from 1 to 100 nm and wherein the hydrophilic coating has a thickness, in particular an average thickness, in the region of max. 500 nm, preferably of max. 300 nm and especially of max. 100 nm.

10. Analytical aid according to one of Claims 1 to 9, wherein the surface of the hydrophilic coating has a contact angle with water of less than 50°, preferably measured according to DIN 55660.2.

11. Analytical aid according to one of Claims 1 to 10, wherein the analytical aid is selected from the group consisting of a needle element, in particular a lancet, a capillary, in particular a capillary gap, a cannula, a testing element for detecting at least one analyte in a body fluid and a distributing element for distributing a sample of a body fluid that has been collected, in particular a spreading network, optionally coated with a metal and/or a metal alloy and/or a metal oxide and/or a mixed metal oxide, or a plastic film, optionally coated with a metal and/or a metal alloy and/or a metal oxide and/or a mixed metal oxide.

12. Analytical aid according to one of Claims 1 to 11, wherein the analytical aid is a needle element (112) of a sampling device (110) for taking a sample of body fluid.

13. Method of production of an analytical aid, comprising a surface that is coated at least partially with a hydrophilic coating, wherein the method comprises:
(a) providing the analytical aid,
(b) coating the analytical aid by bringing the analytical aid into contact with a mixture G comprising at least one dispersant and nanoparticles with silica structure, which have an average particle size, determined according to DIN ISO 22412:2008, in the range from 1 to 500 nm,
wherein the nanoparticles comprise groups of structure (I) or (II) 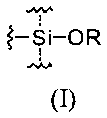 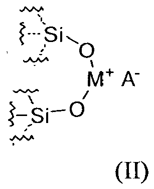 wherein in each of the groups of structure (I), independently of one another, R is selected from the group consisting of H, a metal-containing ion, 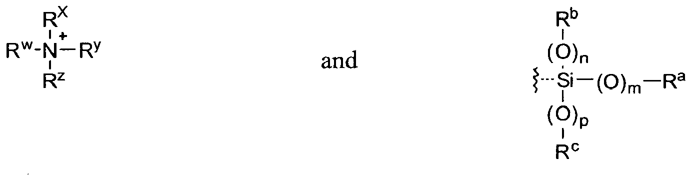 and wherein R^{w}, R^{x}, R^{y} and R^{z}, independently of one another, are selected from H and alkyl,
and wherein R^{a}, R^{b} and R^{c}, independently of one another, are optionally substituted residues, selected from the group consisting of H, alkyl, aryl, heteroaryl, cycloalkyl, cycloheteroalkyl, alkenyl and alkoxyalkyl,
and wherein n, m and p, independently of one another, are 0 or 1,
and wherein M⁺ is a metal ion and A- is a physiologically compatible anion,
(c) drying the analytical aid obtained according to (b), and
(d) optionally sterilizing the analytical aid obtained according to (c),
wherein the bringing into contact in (b) preferably takes place by dip coating and/or spray coating and/or contact coating, and wherein the analytical aid and/or the surface of the analytical aid preferably consists at least partially of a metal and/or a metal alloy and/or a metal oxide and/or a mixed metal oxide and/or a metallic mixed oxide.

14. Method according to Claim 13, wherein the surface of the analytical aid to be coated is treated before step (b) with at least one etchant and/or with plasma.

15. Method according to Claim 13 or 14, wherein the at least one dispersant is water and wherein mixture G preferably has a pH in the range from 2 to 10.

16. Method according to one of Claims 13 to 15, wherein the nanoparticles comprise groups of structure (I), and wherein 10 to 40% of all groups R are

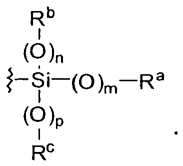

17. Sampling device (110) for taking a sample of body fluid, containing at least one analytical aid according to one of the preceding claims relevant to an analytical aid, with the proviso that the analytical aid is selected from the group consisting of a needle element (112) and a testing element.

18. Use of nanoparticles with silica structure and an average particle size, determined according to DIN ISO 22412:2008, in the range from 1 to 500 nm as hydrophilic coating (122) for a surface of an analytical aid, more preferably as hydrophilic coating (122) for a needle element (112) of a sampling device (110) for taking a sample of body fluid, wherein the nanoparticles comprise groups of structure (I) or (II) 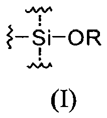 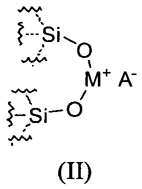 wherein in each of the groups of structure (I), independently of one another, R is selected from the group consisting of H, a metal-containing ion, 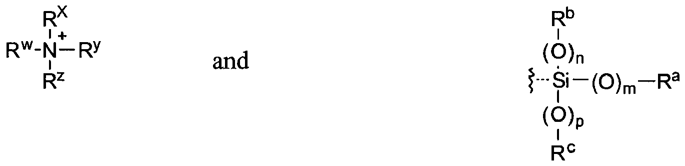 and wherein R^{w}, R^{x}, R^{y} and R^{z}, independently of one another, are selected from H and alkyl,
and wherein R^{a}, R^{b} and R^{c}, independently of one another, are optionally substituted residues, selected from the group consisting of H, alkyl, aryl, heteroaryl, cycloalkyl, cycloheteroalkyl, alkenyl and alkoxyalkyl,
and wherein n, m and p, independently of one another, are 0 or 1,
and wherein M⁺ is a metal ion and A- is a physiologically compatible anion.

## Revendications

1. Adjuvant analytique comprenant une surface enduite au moins en partie avec un revêtement hydrophile, **caractérisé en ce que**
le revêtement hydrophile contient des nanoparticules possédant une structure de dioxyde de silicium et une granulométrie moyenne déterminée conformément à la norme DIN ISO 22412:2008, dans la plage de 1 à 500 nm, et les nanoparticules comprennent des groupes possédant la structure (I) ou (II) 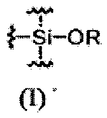 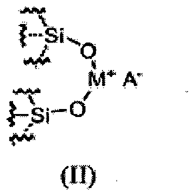 dans lequel, dans chacun des groupes possédant la structure (I), R est choisi, indépendamment l'un de l'autre, parmi le groupe constitué par un atome d'hydrogène, un ion contenant un métal, 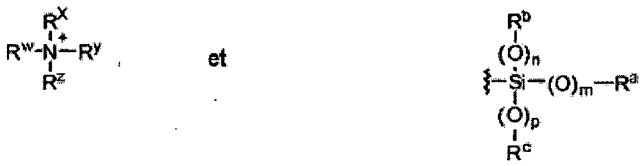 et dans lequel R^{w}, R^{x}, R^{y} et R^{z}, sont choisis, indépendamment l'un de l'autre, parmi un atome d'hydrogène et un groupe alkyle,
et dans lequel les résidus R^{a}, R^{b} et R^{c} sont des groupes substitués de manière facultative choisis, indépendamment l'un de l'autre, parmi le groupe constitué par un atome d'hydrogène, un groupe alkyle, un groupe aryle, un groupe hétéroaryle, un groupe cycloalkyle, un groupe cyclohétéroalkyle, un groupe alcényle et un groupe alcoxyalkyle ;
et dans lequel n, m et p, sont égaux, indépendamment l'un de l'autre, à 0 ou 1 ;
et dans lequel M⁺ représente un ion métallique et A⁻ représente un anion physiologiquement acceptable.

2. Adjuvant analytique selon la revendication 1, dans lequel R est choisi parmi le groupe constitué par un atome d'hydrogène, un ion de métal alcalino-terreux, un ion de métal alcalin 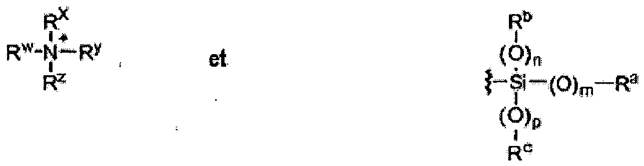 et dans lequel M⁺ = Al⁺.

3. Adjuvant analytique selon la revendication 1 ou 2, dans lequel la structure de dioxyde de silicium des nanoparticules présente au moins un atome étranger choisi parmi le groupe constitué par Sn, Ti, Zr, Ge, In, Ga, Al et B, de préférence dans lequel l'atome étranger représente un atome d'aluminium.

4. Adjuvant analytique selon l'une quelconque des revendications 1 à 3, dans lequel les nanoparticules présentent une granulométrie moyenne dans la plage de 1 à 100 nm, de préférence dans la plage de 1 à 50 nm, de manière plus préférée dans la plage de 3 à 20 nm.

5. Adjuvant analytique selon l'une quelconque des revendications 1 à 4, dans lequel de 10 à 40 % de tous les groupes R représentent un groupe

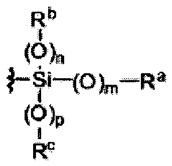

6. Adjuvant analytique selon la revendication 4, dans lequel le groupe répondant à la formule (I) présente une structure répondant à la formule (la) ou (Ib): 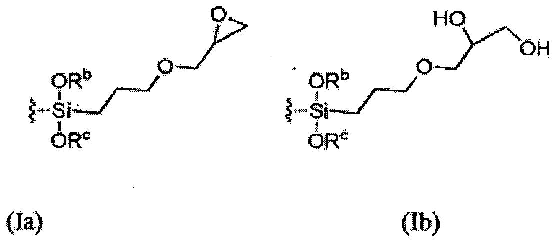 dans laquelle R^{b} et R^{c} sont choisis, indépendamment l'un de l'autre, parmi le groupe constitué par un atome d'hydrogène et un groupe alkyle.

7. Adjuvant analytique selon l'une quelconque des revendications 1 à 5, dans lequel le revêtement hydrophile est constitué par les nanoparticules comprenant une structure de dioxyde de silicium.

8. Adjuvant analytique selon l'une quelconque des revendications 1 à 7, dans lequel la surface de l'adjuvant analytique, enduite au moins en partie avec le revêtement hydrophile, est constituée au moins en partie d'un métal et/ou d'un alliage métallique et/ou d'un oxyde métallique et/ou d'un oxyde métallique mixte.

9. Adjuvant analytique selon l'une quelconque des revendications 1 à 7, dans lequel les nanoparticules présentent une granulométrie moyenne dans la plage de 1 à 100 nm et dans lequel le revêtement hydrophile présente une épaisseur, en particulier une épaisseur moyenne, au maximum de 500 nm, de préférence au maximum de 300 nm et en particulier au maximum de 100 nm.

10. Adjuvant analytique selon l'une quelconque des revendications 1 à 9, dans lequel la surface du revêtement hydrophile présente un angle de contact avec l'eau inférieur à 50°, mesuré de préférence conformément à la norme DIN 55660.2.

11. Adjuvant analytique selon l'une quelconque des revendications 1 à 10, dans lequel l'adjuvant analytique est choisi parmi le groupe constitué par un élément en forme d'aiguille, en particulier une lancette, un capillaire, en particulier une fente capillaire, une canule, un élément de test pour le décèlement d'au moins un analyte dans un liquide corporel, et un élément de distribution pour la distribution d'un échantillon prélevé d'un liquide corporel, en particulier un filet d'étalement enduit de manière facultative avec un métal et/ou avec un alliage métallique et/ou avec un oxyde métallique et/ou avec un oxyde métallique mixte, ou une feuille en matière synthétique enduite de manière facultative avec un métal et/ou avec un alliage métallique et/ou avec un oxyde métallique et/ou avec un oxyde métallique mixte.

12. Adjuvant analytique selon l'une quelconque des revendications 1 à 11, dans lequel l'adjuvant analytique est un élément en forme d'aiguille (112) d'un dispositif de prélèvement d'échantillon (110) pour le prélèvement d'un liquide corporel.

13. Procédé pour la production d'un adjuvant analytique, comprenant une surface qui est enduite au moins en partie avec un revêtement hydrophile, le procédé comprenant le fait de :
(a) procurer l'adjuvant analytique;
(b) enduire l'adjuvant analytique par la mise en contact de l'adjuvant analytique avec un mélange G comprenant au moins un agent de mise en dispersion et des nanoparticules possédant une structure de dioxyde de silicium, qui présentent une granulométrie moyenne déterminée conformément à la norme DIN ISO 22412:2008, dans la plage de 1 à 500 nm ;
dans lequel les nanoparticules comprennent des groupes possédant la structure (I) ou (II) 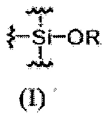 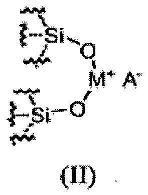 dans lequel, dans chacun des groupes possédant la structure (I), R est choisi, indépendamment l'un de l'autre, parmi le groupe constitué par un atome d'hydrogène, un ion contenant un métal, 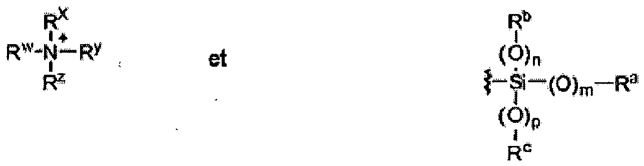 et dans lequel R^{w}, R^{x}, R^{y} et R^{z}, sont choisis, indépendamment l'un de l'autre, parmi un atome d'hydrogène et un groupe alkyle,
et dans lequel les résidus R^{a}, R^{b} et R^{c} sont des groupes substitués de manière facultative choisis, indépendamment l'un de l'autre, parmi le groupe constitué par un atome d'hydrogène, un groupe alkyle, un groupe aryle, un groupe hétéroaryle, un groupe cycloalkyle, un groupe cyclohétéroalkyle, un groupe alcényle et un groupe alcoxyalkyle ;
et dans lequel n, m et p, sont égaux, indépendamment l'un de l'autre, à 0 ou 1 ;
et dans lequel M⁺ représente un ion métallique et A⁻ représente un anion physiologiquement acceptable ;
(c) sécher l'adjuvant analytique que l'on obtient conformément à (b) ; et
(d) de manière facultative, stériliser l'adjuvant analytique obtenu conformément à (c) ;
dans lequel la mise en contact dans (b) a lieu via de préférence une enduction par immersion et/ou une enduction par pulvérisation et/ou une enduction par contact, et dans lequel l'adjuvant analytique et/ou la surface de l'adjuvant analytique sont constitués de préférence au moins en partie d'un métal et/ou d'un alliage métallique et/ou d'un oxyde métallique et/ou d'un oxyde métallique mixte et/ou d'un oxyde mixte métallique.

14. Procédé selon la revendication 13, dans lequel la surface à enduire de l'adjuvant analytique est soumise à un traitement avec au moins un agent de gravure et/ou avec un plasma avant l'étape (b).

15. Procédé selon la revendication 13 ou 14, dans lequel ledit au moins un agent de mise en dispersion est de l'eau et dans lequel le mélange G présente une valeur de pH dans la plage de 2 à 10.

16. Procédé selon l'une quelconque des revendications 13 à 15, dans lequel les nanoparticules comprennent des groupes répondant à la structure (I) et dans lequel de 10 à 40 % de tous les groupes R représentent un groupe

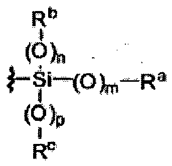

17. Dispositif de prélèvement d'échantillon (110) pour le prélèvement d'un liquide corporel, contenant au moins un adjuvant analytique selon l'une quelconque des revendications précédentes concernant un adjuvant analytique, avec cette mesure que l'adjuvant analytique est choisi parmi le groupe constitué par un élément en forme d'aiguille (112) et un élément de test.

18. Utilisation de nanoparticules possédant une structure de dioxyde de silicium et une granulométrie moyenne déterminée conformément à la norme DIN ISO 22412:2008, dans la plage de 1 à 500 nm, à titre de revêtement hydrophile (122) pour une surface d'un adjuvant analytique, de manière plus préférée à titre de revêtement hydrophile (122) pour un élément en forme d'aiguille (112) d'un dispositif de prélèvement d'échantillon (110) pour le prélèvement d'un liquide corporel, les nanoparticules comprenant des groupes possédant la structure (I) ou (II) 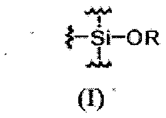 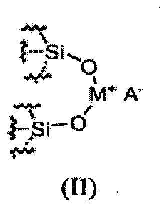 dans lequel, dans chacun des groupes possédant la structure (I), R est choisi, indépendamment l'un de l'autre, parmi le groupe constitué par un atome d'hydrogène, un ion contenant un métal, 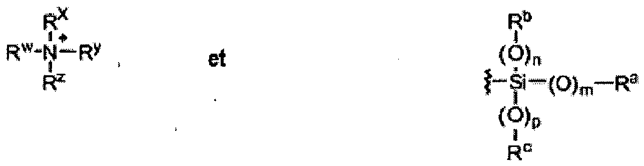 et dans lequel R^{w}, R^{x}, R^{y} et R^{z}, sont choisis, indépendamment l'un de l'autre, parmi un atome d'hydrogène et un groupe alkyle,
et dans lequel les résidus R^{a}, R^{b} et R^{c} sont des groupes substitués de manière facultative choisis, indépendamment l'un de l'autre, parmi le groupe constitué par un atome d'hydrogène, un groupe alkyle, un groupe aryle, un groupe hétéroaryle, un groupe cycloalkyle, un groupe cyclohétéroalkyle, un groupe alcényle et un groupe alcoxyalkyle ;
et dans lequel n, m et p, sont égaux, indépendamment l'un de l'autre, à 0 ou 1 ;
et dans lequel M⁺ représente un ion métallique et A- représente un anion physiologiquement acceptable.
